(19) Europäisches Patentamt
European Patent Office
Office européen des brevets

(11) EP 3 429 346 B1

(12) **FASCICULE DE BREVET EUROPEEN**

(45) Date de publication et mention de la délivrance du brevet:
**22.04.2020 Bulletin 2020/17**

(21) Numéro de dépôt: **17710579.8**

(22) Date de dépôt: **17.03.2017**

(51) Int Cl.:
*A01N 25/02* (2006.01) *A01N 25/30* (2006.01)
*A01N 35/02* (2006.01) *A01P 1/00* (2006.01)
*A01P 7/00* (2006.01) *A61K 8/02* (2006.01)
*A61K 8/06* (2006.01) *A61K 8/37* (2006.01)
*A61K 8/60* (2006.01) *A61K 8/86* (2006.01)
*A61Q 19/00* (2006.01)

(86) Numéro de dépôt international:
**PCT/EP2017/056464**

(87) Numéro de publication internationale:
**WO 2017/158193 (21.09.2017 Gazette 2017/38)**

(54) **COMPOSITION COMPRENANT UN MEL, UN ESTER METHYLIQUE D'ACIDE GRAS ET UN TENSIOACTIF NON-IONIQUE DE HLB SUPERIEUR OU EGAL A 12**

ZUSAMMENSETZUNG ENTHALTEND EINEN MEL, EINEN FETTSÄUREMETHYLESTER UND EIN NICHTIONISCHES TENSID MIT EINEM HLB-WERT GRÖSSER ODER GLEICH 12

COMPOSITION COMPRISING A MEL, A FATTY ACID METHYL ESTER AND A NON-IONIC SURFACTANT HAVING AN HLB VALUE GREATER THAN OR EQUAL TO 12

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.03.2016 FR 1652280**

(43) Date de publication de la demande:
**23.01.2019 Bulletin 2019/04**

(73) Titulaire: **Oleon N.V.**
**9940 Evergem (Ertvelde) (BE)**

(72) Inventeurs:
- **RANDU, Marion**
**60600 Clermont (FR)**
- **HERY, Sylvie**
**60880 Jaux (FR)**
- **RAVIER, Pierre**
**60200 Compiegne (FR)**
- **DEPREY, Sophie**
**60280 Margny-Les-Compiegne (FR)**

(74) Mandataire: **Santarelli**
**49, avenue des Champs-Elysées**
**75008 Paris (FR)**

(56) Documents cités:
**WO-A1-2013/054194**

- **TOKUMA FUKUOKA ET AL: "Application of Yeast Glycolipid Biosurfactant, Mannosylerythritol Lipid, as Agrospreaders", JOURNAL OF OLEO SCIENCE, vol. 64, no. 6, 1 janvier 2015 (2015-01-01), pages 689-695, XP055280365, JP ISSN: 1345-8957, DOI: 10.5650/jos.ess15017**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 429 346 B1

## Description

La présente invention se rapporte à une composition émulsionnable et à une émulsion la comprenant. La présente invention concerne également un procédé de préparation de la composition et de l'émulsion selon l'invention, et l'utilisation de la composition selon l'invention, en particulier dans la préparation d'une émulsion. WO 2013/054194 décrit une composition biodégradable pour la préparation de concentrés émulsifiables de pesticides et ses émulsions.

Dans le cadre de la présente demande, par « composition émulsionnable », on entend une composition qui peut être portée à l'état d'émulsion, notamment lors de la mise en contact avec de l'eau. Les compositions émulsionnables trouvent une application dans de nombreux domaines. Il est notamment connu d'utiliser des compositions émulsionnables dans l'industrie cosmétique et dans l'industrie agricole, notamment pour la protection des plantes. Dans l'industrie agricole, les compositions émulsionnables, également appelées concentrés émulsionnables, sont généralement utilisés dans la préparation de bouillies phytosanitaires, telles que sous forme d'émulsions phytosanitaires, comme par exemple des émulsions herbicide, fongicide, insecticide, algicide ou encore stimulatrice des défenses des plantes.

Outre leur émulsionnabilité ("emulsifiability" ou capacité à être porté à l'état d'émulsion), les compositions émulsionnables peuvent posséder d'autres propriétés. A titre d'exemple, dans la cadre d'une utilisation phytosanitaire, une composition émulsionnable peut également posséder une propriété pénétrante, humectante et/ou mouillante. Une composition émulsionnable présentant une propriété mouillante favorise l'étalement et la rétention de l'émulsion la comprenant sur les cultures. Il en résulte, d'une part, une réduction de la quantité d'émulsion phytosanitaire à pulvériser sur les cultures, et d'autre part, une amélioration de l'efficacité des émulsions phytosanitaires. L'utilisation de compositions émulsionnables ayant des propriétés mouillantes permet ainsi une réduction des efforts et des coûts associés au traitement des cultures.

En outre, quels que soient les types d'industrie dans lesquels elles sont utilisées, il est préférable que les compositions émulsionnables soient respectueuses de l'environnement et de moindre toxicité pour les opérateurs les utilisant. Cela est particulièrement important lorsqu'elles sont utilisées dans la préparation d'émulsions phytosanitaires, car celles-ci sont généralement déversées en quantité non négligeable sur les cultures, de sorte qu'il est préférable, voire nécessaire, que les compositions émulsionnables soient écologiquement avantageuses, et notamment biodégradables.

Plus particulièrement, il serait intéressant de développer des compositions émulsionnables :

- possédant une excellente émulsionnabilité,
- permettant l'obtention d'émulsions stables,
- possédant une bonne capacité à augmenter le pouvoir mouillant des émulsions les comprenant, telles que des émulsions phytosanitaires, et
- qui seraient en outre respectueuses de l'environnement.

Le travail de l'inventeur a permis de mettre en évidence qu'une composition spécifique présentait l'ensemble des propriétés avantageuses décrites ci-dessus.

L'invention concerne donc une composition comprenant :

- entre 75 et 99% en poids d'au moins un ester méthylique ou éthylique d'acide gras,
- entre 0,01 et 20% en poids d'au moins un lipide de mannosylérythritol, et
- entre 0,01 et 20% en poids d'au moins un tensioactif non-ionique de HLB supérieur ou égal à 12,

les pourcentages en poids étant indiqués par rapports au poids total de la composition.

On notera que dans le cadre de la présente demande, et sauf stipulation contraire, les gammes de valeurs indiquées s'entendent bornes incluses.

La composition selon l'invention comprend entre 75 et 99% en poids d'au moins un ester méthylique ou éthylique d'acide gras, par rapport au poids total de la composition.

Avantageusement, le ou les ester(s) méthylique(s) ou éthylique(s) d'acide(s) gras compris dans la composition selon l'invention sont choisis parmi l'oléate de méthyle, l'oléate d'éthyle, les esters méthyliques ou éthyliques d'acide gras d'huile de colza, de soja, d'olive, de tournesol, de ricin, de palme et/ou de lin, ou leurs mélanges.

De préférence, la quantité totale d'ester(s) méthylique(s) ou éthylique(s) d'acide(s) gras dans la composition selon l'invention est comprise entre 80 et 98% en poids, par rapport au poids total de la composition.

Par quantité totale d'ester(s) méthylique(s) ou éthylique(s) d'acide(s) gras présente dans la composition, on entend la quantité de molécule(s) d'ester(s) méthylique(s), respectivement d'ester(s) éthylique(s), d'acide(s) gras présente dans ladite composition.

De préférence, la quantité totale d'ester(s) méthylique(s) ou éthylique(s) d'acide(s) gras dans la composition selon l'invention est comprise entre 85 et 96% en poids, par rapport au poids total de la composition.

De préférence, le ou les ester(s) méthylique(s) ou éthylique(s) d'acide(s) gras compris dans la composition selon l'invention sont des esters méthyliques ou éthylique(s) d'acides gras comportant une chaine carbonée ayant entre 8 et 24 atomes de carbones, plus préférentiellement entre 12 et 20 atomes de carbone, encore plus préférentiellement entre 16 et 18 atomes de carbone.

De préférence, dans la composition selon l'invention, le ou les ester(s) méthylique(s) ou éthylique(s) d'acide(s) gras sont des esters méthyliques ou éthyliques d'acides gras d'huile de colza, de soja et/ou d'olive, plus préférentiellement de colza et/ou d'olive.

De préférence, la composition selon l'invention comprend au moins un ester méthylique d'acide gras.

La composition selon l'invention comprend également entre 0,01 et 20% en poids d'au moins un lipide de mannosylérythritol, par rapport au poids total de la composition.

Selon l'invention, un « lipide de mannosylérythritol » (également appelé « MEL ») est un tensioactif appartenant à la classe des glycolipides. Plus particulièrement, un MEL est une molécule amphiphile dont la partie hydrophile est formée d'un résidu mannosylérythritol, et dont la partie hydrophobe est formée par au moins un acide gras.

Plus particulièrement, par « MEL », on désigne une molécule présentant la formule générale (I) suivante :

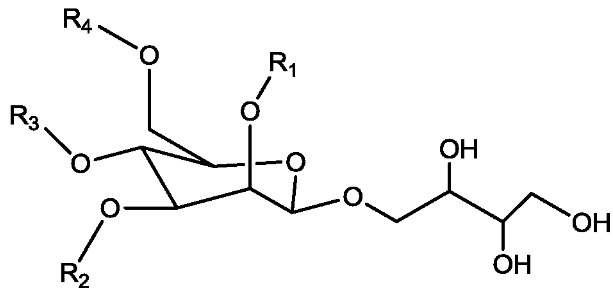

(I)

dans laquelle :

- $R_1$ et $R_2$, identiques ou différents, représentent un acide gras insaturé ou saturé, et
- $R_3$ et $R_4$, identiques ou différents, représentent un groupement acétyl ou un atome d'hydrogène.

De préférence, dans la présente invention, par « MEL », on désigne une molécule de formule (II) suivante :

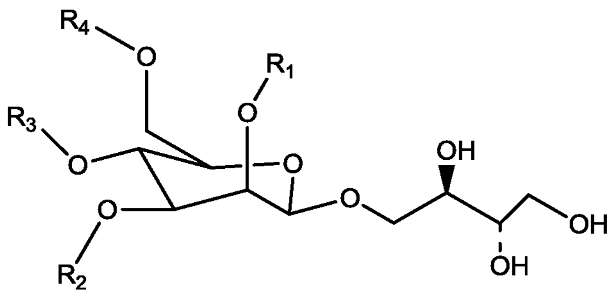

(II)

dans laquelle :

- $R_1$ et $R_2$, identiques ou différents, représentent un acide gras insaturé ou saturé, et
- $R_3$ et $R_4$, identiques ou différents, représentent un groupement acétyle ou un atome d'hydrogène.

Les formules (I) et (II) ci-avant peuvent représenter plusieurs molécules, chaque molécule étant donc un MEL. Par « MELs », on désigne au moins deux molécules de formules (I), et plus particulièrement de formule (II), différentes.

Les MELs sont généralement classés en quatre classes de molécules, notées de A à D, selon leur degré d'acétylation en $R_3$ et $R_4$. La classe des MELs-A comporte des molécules de formules (I) ou (II) présentant deux groupes acétyles en $R_3$ et $R_4$. La classe des MELs-B et la classe des MELs-C comportent des molécules de formules (I) ou (II) présentant un seul groupe acétyle en $R_4$ et $R_3$ respectivement. Enfin, la classe des MELs-D comporte des molécules de formules (I) ou (II) ne présentant pas de groupe acétyle ($R_3$=$R_4$=H).

Outre de par leur degré d'acétylation, les MELs peuvent varier dans leur structure, de par la nature des acides gras qui composent leur partie hydrophobe. Cette variation est généralement fonction du procédé mis en oeuvre pour

l'obtention des MELs.

Les MELs sont généralement obtenus par des procédés mettant en oeuvre la culture de champignons, et plus particulièrement de levures.

Avantageusement, les MELs visés par la présente demande sont obtenus par un procédé de fermentation, comprenant les étapes suivantes :

- la culture d'une souche de champignon et plus particulièrement d'une souche de levure en présence d'une source de carbone pour obtenir des MELs; et
- la récupération des MELs ainsi obtenus.

Les souches à partir desquelles il est possible d'obtenir des MELs sont bien connues de l'homme du métier. A titre d'exemple, il est connu d'utiliser des souches de champignons du genre *Pseudozyma* ou du genre *Ustilago,* pour l'obtention de MELs.

Avantageusement, les souches utilisées dans le procédé de fermentation décrit ci-avant, permettant l'obtention de MELs, sont des souches de champignons appartenant au genre *Pseudozyma.* De préférence, la souche est *Pzeudozyma antartica* ou *Pzeudozyma aphidis.*

De telles souches sont usuellement cultivées en réacteur dans un milieu comportant du glucose, de l'eau et/ou des sels (tel que le sulfate de magnésium, le phosphate de monopotassium et/ou le nitrate d'ammonium).

Avantageusement, les différents composants du milieu (glucose et souches en particulier) sont stérilisés séparément avant introduction dans le réacteur.

La température du milieu est de préférence comprise entre 20 et 35°C, plus préférentiellement entre 25 et 30 °C.

Avantageusement, la source de carbone permettant la production de MELs par la souche est une huile, telle qu'une huile végétale. De préférence, la source de carbone est une huile de soja ou encore plus préférentiellement une huile de colza. Ces huiles sont particulièrement riches en acides gras comportant une chaine carbonée à 18 atomes de carbone, tels qu'en acide oléique, linoléique et/ou linolénique, ainsi que, dans une moindre mesure, en acides gras comportant une chaine carbonée à 16 atomes de carbone, tel qu'en acide palmitique.

La récupération des MELs subséquente à l'étape de culture peut comporter une étape de séparation des MELs des autres composants du milieu. Cette étape peut se faire par des méthodes de séparation classiques connues de l'homme du métier.

Avantageusement, la récupération des MELs peut comprendre une ou plusieurs des méthodes de séparation suivantes :

- la décantation ;
- l'évaporation de l'eau ou le séchage ;
- la filtration; et/ou
- la centrifugation.

Avantageusement, la quantité totale de MEL(s) dans la composition selon l'invention est comprise entre 0,1 et 10% en poids, par rapport au poids total de la composition.

Par quantité totale de MEL(s) présente dans la composition, on entend la quantité de molécule(s) de MEL(s) de formules (I) ou (II) présente dans ladite composition.

De préférence, la quantité totale de MEL(s) est comprise entre 0,5 et 6% en poids, plus préférentiellement entre 0,8 et 3% en poids, par rapport au poids total de la composition.

Favorablement, la composition comprend au moins deux MELs choisis parmi le groupe constitué par MEL-A, MEL-B, MEL-C et MEL-D.

De préférence, la composition selon l'invention comprend des MEL(s)-A, MEL(s)-B, MEL(s)-C et optionnellement des MEL(s)-D, plus préférentiellement des MEL(s)-A, MEL(s)-B, MEL(s)-C et des MEL(s)-D.

Avantageusement, la composition selon l'invention comprend des MELs-A et MELs-B à une teneur comprise entre 50 et 90% en poids, de préférence comprise entre 60 et 85% en poids, les pourcentages en poids étant indiqués par rapport au poids de la quantité totale de MELs.

Avantageusement, la composition selon l'invention comprend des MELs-C à une teneur supérieure ou égale à 5% en poids, de préférence supérieure ou égale à 10% en poids, les pourcentages en poids étant indiqués par rapport au poids de la quantité totale de MELs.

Plus particulièrement, la composition selon l'invention peut comprendre des MELs-A et MELs-B à une teneur comprise entre 60% et 75% en poids, et des MELs-C à une teneur supérieure ou égale à 20% en poids, les pourcentages en poids étant indiqués par rapport au poids de la quantité totale de MELs.

La composition selon l'invention comporte également entre 0,01 et 20% en poids d'au moins un tensioactif non-ionique de HLB supérieur ou égal à 12.

Le HLB permet de définir, et en particulier de chiffrer, l'équilibre entre la partie hydrophile et la partie lipophile d'une molécule de tensioactif, cet équilibre étant lié à la solubilité du tensioactif dans l'eau. La valeur de HLB peut varier de 0 à 20. Plus la valeur de HLB est élevée, plus la solubilité du tensioactif dans l'eau est grande.

Dans le cadre de la présente demande, le calcul du HLB se fait par la méthode de Griffin :

$$HLB = 20 \times \frac{\text{Masse moléculaire partie hydrophile}}{\text{Masse moléculaire de la molécule}}$$

Avantageusement, la quantité totale de tensioactif(s) non-ionique(s) de HLB supérieur ou égal à 12 dans la composition selon l'invention est comprise entre 0,1 et 15% en poids, par rapport au poids total de la composition.

Par quantité totale de tensioactif(s) non-ionique(s) de HLB supérieur ou égal à 12 présente dans la composition, on entend la quantité de molécule(s) de tensioactif(s) non-ionique(s) de HLB supérieur ou égal à 12 présente dans ladite composition.

De préférence, la quantité totale de tensioactif(s) non-ionique(s) de HLB supérieur ou égal à 12 est comprise entre 0,5 et 10% en poids, plus préférentiellement entre 0,8 et 8% en poids, encore plus préférentiellement entre 1 et 5% en poids, par rapport au poids total de la composition.

De préférence, le ou les tensioactif(s) non-ionique(s) de HLB supérieur ou égal à 12 compris dans la composition selon l'invention est/sont choisi(s) parmi le groupe constitué par :

- les polysorbates (esters d'acides gras et de sorbitol éthoxylés), tel que le polysorbate 20, le polysorbate 60 et/ou le polysorbate 80,
- les alkylpolyglucosides, tels que ceux ayant des chaines alkyles comportant entre 4 et 16 atomes de carbones,
- les esters d'acides gras et de polyéthylène glycol (« Polyethyleneglycol fatty acid esters »), et,
- les esters d'acides gras et de polyglycérol (« Polygycerol fatty acid esters »).

Avantageusement, le ou les tensioactif(s) non-ionique(s) compris dans la composition selon l'invention ont un HLB (« Hydrophilic-Lipophilic Balance ») compris entre 12 et 20, de préférence entre 12 et 18, plus préférentiellement entre 12 et 16, encore plus préférentiellement entre 12 et 14.

De préférence, le ou les tensioactif(s) non-ionique(s) compris dans la composition selon l'invention ont un HLB supérieur ou égal à 12,5, plus préférentiellement supérieur ou égal à 13.

La composition selon l'invention est émulsionnable. Plus particulièrement, la composition selon l'invention a une excellente émulsionnabilité, c'est-à-dire que lorsqu'elle est ajoutée à de l'eau, la composition selon l'invention permet la formation d'une émulsion :

- uniforme (complète), et
- de façon spontanée.

Par « de façon spontanée », on entend que seule une faible agitation est nécessaire pour obtenir une émulsion. A titre d'exemple de faible agitation, le contenant dans lequel la composition selon l'invention et l'eau sont ajoutés peut être retourné manuellement de sorte à pivoter d'un angle de 180°C, puis ramené à sa position initiale, le tout en environ deux secondes, conformément à ce qui est indiqué dans l'étape (i) de la norme CIPAC MT 36.3 (« CIPAC method 2000. Prepared by the German Formulation Panel (DAPF). Chairman : G Menschel. »).

Dans le cadre de la présente demande, toute référence faite à une norme est une référence à la norme en vigueur à la date de dépôt.

Avantageusement, l'émulsionnabilité de la composition selon l'invention est caractérisée conformément à l'étape i) de la norme CIPAC MT 36.3.

L'émulsionnabilité de la composition selon l'invention est plus amplement décrite dans l'Exemple 2.

En outre, lorsqu'une émulsion est préparée à partir de la composition selon l'invention, celle-ci est stable dans le temps.

De plus, il a été mis en évidence par les inventeurs que, de façon surprenante, une émulsion comprenant la composition selon l'invention a un pouvoir mouillant élevé, et possède une couleur blanche particulièrement favorable à son utilisation dans divers domaines, comme par exemple en cosmétique. Ces caractéristiques sont plus amplement décrites ci-après.

Avantageusement, la composition selon l'invention comprend entre 0,1 et 20% en poids d'au moins un alcool ayant un nombre d'atomes de carbone compris entre 1 et 16, par rapport au poids total de la composition.

Par « alcool », on vise plus particulièrement un alcool linéaire ou ramifié. Par « linéaire ou ramifié », on exclut spécifiquement les alcools cycliques. Plus particulièrement encore, l'alcool est un alcool constitué d'une chaîne hydrocarbonée, linéaire ou ramifiée, substituée par un ou plusieurs groupement(s) hydroxyle(s) (OH). Par « chaîne hydrocarbonée », on entend une chaîne constituée uniquement d'atomes de carbone et d'hydrogène, la chaîne hydrocarbonée comportant alors entre 1 et 16 atomes de carbone. En d'autres termes, l'alcool ne comporte pas d'hétérosubstituant autre que le ou les groupement(s) hydroxyle(s).

Le ou les alcool(s) compris dans la composition selon l'invention permettent encore d'améliorer la stabilité et le pouvoir mouillant des émulsions formées à partir de cette composition.

De préférence, la quantité totale d'alcool(s) dans la composition selon l'invention est comprise entre 0,5 et 10% en poids, par rapport au poids total de la composition.

Par quantité totale d'alcool(s) présente dans la composition, on entend la quantité de molécule(s) d'alcool(s) présente dans ladite composition, les molécules d'alcool étant telles que définies ci-avant.

De préférence, la quantité totale d'alcool(s) est comprise entre 0,8 et 8% en poids, plus préférentiellement entre 1 et 5% en poids, par rapport au poids total de la composition.

Avantageusement, l'alcool est saturé.

Le ou les alcool(s) sont donc linéaire(s) ou ramifié(s). Comme alcool linéaire, on peut citer l'heptanol (ou l'heptan-1-ol), l'octanol (également nommé 15 octan-1-ol ou alcool caprylique), l'alcool laurique ou le nonanol (ou nonan-1-ol ou alcool pélargonique). Comme alcool ramifié, on peut citer l'octan-2-ol, le 2-éthyl-hexanol, le 7-méthyl-octan-1-ol ou le 6-méthyl-pentan-1-ol.

Avantageusement le ou les alcool(s) compris dans la composition selon l'invention ont une chaîne hydrocarbonée comportant un nombre d'atomes de carbone compris entre 4 et 14, préférentiellement entre 6 et 12.

De préférence, le ou les alcool(s) sont choisi(s) parmi les alcools ayant une chaîne hydrocarbonée comportant 8 à 10 atomes de carbone ou leurs mélanges. En particulier, il s'agit de l'octanol, l'octan-2-ol, le 2- éthyl-hexanol et/ou l'alcool laurique, de préférence l'octan-2-ol.

Avantageusement, l'alcool peut être obtenu à partir de ressources renouvelables, telles qu'à partir de graisses animales ou d'huiles végétales.

De manière particulièrement préférée, le tensioactif non-ionique de HLB supérieur ou égal à 12 présent dans la composition selon l'invention est un ester d'acide gras et de polyéthylène glycol.

Dans le cadre de la présente demande, par « polyéthylène glycol », également appelé « PEG », on entend un polymère d'oxydes d'éthylène ayant une masse molaire inférieure à 20000 $g.mol^{-1}$.

En effet, lorsque la composition selon l'invention comprend un ester d'acide gras et de polyéthylène glycol, une émulsion préparée à partir de cette composition aura un pouvoir mouillant particulièrement élevé. Cet effet est plus amplement décrit dans l'Exemple 5.

Avantageusement, le polyéthylène glycol compris dans l'ester d'acide gras et de polyéthylène glycol a une masse molaire comprise entre 200 et 4000 $g.mol^{-1}$, de préférence comprise entre 300 et 1400 $g.mol^{-1}$, plus préférentiellement comprise entre 400 et 800 $g.mol^{-1}$.

Avantageusement, l'acide gras compris dans l'ester d'acide gras et de polyéthylène glycol a une chaine carbonée comprenant entre 8 et 24 atomes de carbones, de préférence, entre 16 et 20 atomes de carbones.

Un ester d'acide gras et de polyéthylène glycol particulièrement préféré selon l'invention est le mono-oléate de polyéthylèneglycol-600, tel que celui commercialisé par OLEON NV sous la marque RADIA® 7404.

Dans toute la présente demande, lorsqu'un nombre est indiqué derrière le terme « polyéthylène glycol- » ou « PEG- », ce nombre correspond à la masse molaire dudit polyéthylène glycol.

Avantageusement, la composition selon l'invention comprend en outre au moins un acide gras libre et/ou au moins un triglycéride.

Par « acide gras libre », on entend toute molécule d'acide gras qui n'est pas liée à une autre molécule. Par « acide gras », on entend toute molécule d'acide gras liée à une autre molécule, par exemple lorsque cette molécule d'acide gras est présente dans un triglycéride ou dans un MEL.

Le au moins un acide gras libre et/ou au moins un triglycéride peuvent avoir été introduits concomitamment avec le au moins un MEL.

En effet, en fonction du procédé d'obtention des MELs, tel que le procédé de fermentation décrit ci-avant, et en particulier en fonction de la ou des méthode(s) de séparation mise(s) en oeuvre dans l'étape de récupération, ceux-ci peuvent comporter un ou plusieurs acide(s) gras libre(s) et/ou triglycéride(s).

Par exemple, la quantité d'acide(s) gras libre(s) et/ou de triglycéride(s) présente dans la composition selon l'invention peut être comprise entre 0,001 et 15% en poids, de préférence entre 0,01 et 10% en poids, par rapport au poids total de la composition.

Plus particulièrement, la composition comporte au moins un acide gras libre et au moins un triglycéride. Dans ce cas, la quantité d'acide(s) gras libre(s) et de triglycéride(s) présente dans la composition selon l'invention peut être comprise entre 0,001 et 15% en poids, de préférence entre 0,01 et 10% en poids, plus préférentiellement entre 0,1 et 5% en poids, par rapport au poids total de la composition.

Avantageusement, le ou les acide(s) gras libre(s) comporte(nt) une chaine carbonée comportant entre 14 et

24 atomes de carbone, de préférence 16 ou 18 atomes de carbone.

Avantageusement, le ou les triglycéride(s) comportent des acides gras comportant une chaine carbonée comportant entre 14 et 24 atomes de carbones, de préférence 16 ou 18 atomes de carbone.

Plus particulièrement, dans la présente demande, et en particulier dans les exemples, lorsque les MELs, à l'issue de l'étape de récupération, comportent au moins un acide gras libre, au moins un triglycéride, de l'eau et/ou des souches, ce mélange est appelé « mélange de MELs ».

Dans ce cas, le ou les acide(s) gras libre(s) et/ou triglycéride(s) peut/peuvent être issu(s) de l'huile résiduelle présente avec le ou les MEL(s) à la fin du procédé de fermentation décrit ci-avant, ladite huile résiduelle étant l'huile mis en oeuvre en tant que source de carbone dans le procédé de fermentation, qui n'a pas été utilisée par les souches. De plus, le ou les acide(s) gras libre(s) libres peut/peuvent être issu(s) du métabolisme, par les souches, des triglycérides compris dans l'huile mise en oeuvre en tant que source de carbone dans ledit procédé.

En outre, selon le procédé d'obtention des MELs, tel que le procédé de fermentation décrit ci-avant, et en particulier selon la ou les méthode(s) de séparation mise(s) en oeuvre dans l'étape de récupération, les MELs peuvent également comporter de l'eau et des souches de champignons, plus particulièrement des souches de levures.

Selon un mode de réalisation préféré de la composition selon l'invention, celle-ci comporte un mélange de MELs présentant les caractéristiques suivantes :

- une teneur en MELs supérieure ou égale à 40% en poids, de préférence supérieure ou égale à 50% en poids, plus préférentiellement supérieure ou égale à 55% en poids ;
- une teneur en autre composants inférieure ou égale à 60% en poids, de préférence inférieure ou égale à 50% en poids, plus préférentiellement inférieure ou égale à 45% en poids (dont acides gras libres, triglycérides, eau et/ou souches),

les pourcentages en poids étant donnés par rapport au poids total du mélange de MELs.

Avantageusement, dans ce mode de réalisation préféré, la teneur en eau et/ou souches est inférieure à 3% en poids, par rapport au poids total du mélange de MELs.

Ce mélange de MELs peut être notamment obtenu selon le procédé de fermentation décrit ci-avant.

Un exemple de mélange de MELs et son procédé d'obtention est également décrit dans la publication suivante :

- "Downstream processing of mannosylerythritol lipids produced by Pseudozyma aphidis"; Rau et al.; European Journal of Lipids Science and Technology 107 (2005) 373-380.

De préférence, un mélange de MELs comporte des MELs de différentes classes, en général au moins des MELs-A, B et C. Préférentiellement, ce mélange de MELs comporte des MELs-A, B, C et D.

En outre, un mélange de MELs comporte avantageusement des MELs-A et MELs-B à une teneur comprise entre 50 et 90% en poids, de préférence comprise entre 60 et 85% en poids, les pourcentages en poids étant indiqués par rapport au poids de la quantité totale de MELs.

De plus, un mélange de MELs comporte avantageusement des MELs-C à une teneur supérieure ou égale à 5% en poids, de préférence supérieure ou égale à 10% en poids, les pourcentages en poids étant indiqués par rapport au poids de la quantité totale de MELs.

Plus particulièrement, un mélange de MELs peut comporter des MELs-A et des MELs-B à une teneur comprise entre 60% et 75% en poids, et des MELs-C à une teneur supérieure ou égale à 20% en poids, les pourcentages en poids étant indiqués par rapport au poids de la quantité totale de MELs.

De tels mélanges de MELs sont par exemple obtenus à l'aide d'un procédé de fermentation tel que ceux décrits ci-avant.

Il est également possible d'obtenir un mélange de MELs présentant une teneur en MELs supérieure ou égale à 95%, de préférence supérieure ou égale à 98% en poids, par rapport au poids total du mélange de MELs. Ce mélange de MELs peut, par exemple, être obtenu à l'aide du procédé de fermentation décrit ci-avant auquel est ajoutée une étape de purification, à l'issue de l'étape de récupération. Cette étape de purification peut comporter une extraction liquide/liquide et/ou un passage sur un substrat minéral. Le passage sur un substrat minéral peut être une chromatographie, telle qu'une chromatographie d'adsorption sur colonne de silice, réalisée à l'aide de solvants adaptés. De tels solvants sont connus de l'homme du métier.

Selon un mode de réalisation alternatif préféré de la composition selon l'invention, celle-ci peut donc comporter également un mélange de MELs qui présente les caractéristiques suivantes :

- une teneur en MELs supérieure ou égale à 95% en poids, de préférence supérieure ou égale à 98% en poids,

les pourcentages en poids étant donnés par rapport au poids total du mélange de MELs.

En outre, l'étape de purification, suite à l'étape de récupération des MELs, peut être effectuée de sorte à obtenir une classe de MELs ou même un MEL, à une teneur supérieure ou égale à 50%. A titre d'exemple, cette étape de purification peut comporter une extraction liquide/liquide et/ou un passage sur un substrat minéral (tel qu'une chromatographie), tels que définis ci-avant.

Selon un premier mode de réalisation d'une composition selon l'invention, celle-ci est une composition phytosanitaire et comprend un principe actif pesticide.

Avantageusement, le principe actif pesticide est choisi parmi les principes actifs herbicides, fongicides, insecticides, acaricides, algicides, régulateurs de croissance, insectifuges, de bio contrôle et/ou stimulateurs de défense des plantes.

De préférence, le principe actif pesticide est un principe actif herbicide, un principe actif fongicide, un principe actif insecticide et/ou un principe actif stimulateur de défense des plantes.

Avantageusement, la composition phytosanitaire selon l'invention comprend:

- un ou plusieurs principes actifs fongicides tel qu'un carboxamide, une strobilurine, un azole (triazole, imidazole), un composé hétérocyclique (pyridine, pyrimidine, pipérazine, morpholine), un carbamate, une huile essentielle (cinnamaldéhyde, thymol, huile de thé), un micro-organisme (champignons tel que *Gliocladium catenulatum,* levures, bactéries telle que *Bacillus subtilis*) autre que celui pouvant être compris dans le mélange de MELs décrit ci-avant, un polysaccharide (chitosan) et/ou,
- un ou plusieurs principes actifs herbicides tel qu'un inhibiteur de biosynthèse lipidique, un inhibiteur de l'acétolactase synthase (également appelé « inhibiteur d'ALS »), un inhibiteur de photosynthèse, un acétamide, un dérivé d'acides aminés tel qu'un dérivé organophosphoré d'acide aminé (glufosinate ou glyphosate) ou leurs sels (sels d'ammonium du glufosinate, sels de mono ou di ammonium, de potassium, d'isopropylamine du glyphosate), un aryloxyphenoxypoprionate, le bipyridyl, le cyclohexanedione, une dinitroaniline, le diphenyl éther, l'hydroxybenzonitrile, l'imidazolinone, un acide phénoxy acétique, la pyrazine, la pirydine, une sulfonylurée, une triazine, une urée, un carbamate, un acide gras d'origine naturelle ayant une activité herbicide (acide caprylique, acide pélargonique) ou ses dérivés (sels, savons) et/ou,
- un ou plusieurs principes actifs insecticides tel qu'un organo(thio)phosphate, un carbamate, un pyréthrinoïde, un régulateur de croissance des insectes, un agoniste/antagoniste des récepteurs nicotiniques, un antagoniste du GABA, une lactone macrocyclique, le géraniol, l'eugénol, le thymol, l'huile de neem et/ou,
- un ou plusieurs principes actifs stimulateurs de défense des plantes, tel que *Bacillus subtilis,* un dérivé de l'acide jasmonique, un extrait d'algue.

La composition phytosanitaire selon l'invention peut être conservée longtemps avant utilisation, sans que sa teneur en principe actif pesticide ne diminue. En effet, la teneur en principe actif dans la composition selon l'invention diminue très peu au cours du temps, et ce même après plusieurs mois de stockage. Cet effet est plus amplement décrit dans l'Exemple 7.

Avantageusement, le principe actif pesticide est d'origine naturelle. De tels principes actifs pesticides sont généralement appelés principes actifs bio-pesticides ou principes actifs de bio contrôle.

En outre, on notera que le principe actif compris dans la composition phytosanitaire selon l'invention peut avoir à la fois plusieurs des propriétés suivantes: herbicide, fongicide, insecticide, acaricide, régulation de croissance, insectifuge et/ou stimulateur de défense des plantes.

Avantageusement, la quantité de principe actif pesticide est comprise entre 0,1 et 30% en poids, de préférence entre 1 et 20% en poids, plus préférentiellement entre 5 et 15% en poids par rapport au poids total de la composition phytosanitaire.

La présente demande vise plus particulièrement une composition selon l'invention, en combinaison avec du glyphosate ou un de ses sels, et/ou du glufosinate ou un de ses sels, et/ou du cinnamaldéhyde, et/ou *Bacillus subtilis,* et/ou *Gliocladium catenulatum.*

De préférence, la composition selon l'invention est en combinaison avec du glyphosate ou un de ses sels et/ou du glufosinate ou un de ses sels.

Selon un second mode de réalisation d'une composition selon l'invention, celle-ci est une composition cosmétique et comprend principe actif cosmétique.

Avantageusement, la composition cosmétique comprend un ou plusieurs principes actifs cosmétiques choisis parmi :

- un agent hydratant tel que l'huile de jojoba, l'huile d'amande douce, la paraffine, l'huile de germe de blé, le collagène, la pectine, le chitosan, un glycosaminoglycane et/ou,
- un filtre UV organique tel que le PABA, le PARA, un salicylate, un cinnamate, un anthranilate, le benzophenone-3, le butyl méthoxydibenzoyléthane, l'éthylhexyl triazone, le dometrizol trisiloxane, le diéthylhexyl butamido triazone,

le 4-méthylbenzylidène camphor, le bemotrizinol, le diéthylamino hydroxybenzoyl hexyl benzoate, le phényl salicylate, le méthylène bis-benzotriazolyl tetramethylbutylphenol, le benzophenone-1, benzophenone-2, benzophenone-8, le bis-éthylhexyloxyphenol methoxy-phenyl triazine, ou un filtre UV minéral et/ou,
- un agent anti-âge tel qu'un rétinoïde, un $\alpha$- ou $\beta$-hydroxy acide, une vitamine hydrosoluble, le palmitate d'ascorbyle, un céramide, un pseudo céramide, un phospholipide, le cholestérol, un stérol et/ou,
- un agent anti-cellulite tel que l'isobutylméthylxanthine, la théophylline et/ou,
- agent anti-acné tel que le résorcinol, l'acétate de résorcinol, le peroxyde de benzoyle, l'acide salicylique, l'acide azélaïque et/ou,
- un agent raffermissant tel qu'un extrait de plantes (extrait de graines de lin), l'eau de rose et/ou,
- une vitamine telle que la vitamine A, ses dérivés, la vitamine B2, l'acide pantothénique, la vitamine D, la vitamine E.

Avantageusement la quantité de principe actif cosmétique est comprise entre 0,1 et 30% en poids, de préférence entre 0,5 et 20% en poids, plus préférentiellement entre 1 et 15% par rapport au poids total de la composition cosmétique.
En outre, une composition selon l'invention peut être utilisée dans diverses autres applications, tel que dans les pompes à incendies.
L'invention concerne également un procédé de préparation d'une composition selon l'invention, comprenant une étape de mélangeage d'au moins un ester méthylique ou éthylique d'acide gras, d'au moins un lipide de mannosylérythritol et d'au moins un tensioactif non-ionique de HLB supérieur ou égal à 12.
Les compositions selon l'invention sont facilement préparables, par simple mélangeage des composants.
Avantageusement, le mélangeage est réalisé à température ambiante dans les conditions normales de température et de pression (CNTP).
De préférence, pendant le mélangeage, les composants sont chauffés à une température comprise entre 25 et 55 °C, plus préférentiellement entre 30 et 50 °C, encore plus préférentiellement entre 35 et 45 °C.
Le chauffage des composants peut permettre une meilleure homogénéisation de la composition selon l'invention.
Avantageusement, le procédé de préparation d'une composition selon l'invention comprend une étape de mélangeage d'au moins un ester méthylique ou éthylique d'acide gras, d'au moins un lipide de mannosylérythritol, d'au moins un tensioactif non-ionique de HLB supérieur ou égal à 12 et d'au moins un alcool ayant entre 1 et 16 atomes de carbone.
Optionnellement, préalablement au mélangeage, le procédé de préparation d'une composition selon l'invention comprend l'obtention d'au moins un MEL, tel que cela est décrit ci-avant.
Avantageusement, le(s) MEL(s) sont tels que décrits ci-avant et peuvent être obtenus par le procédé de fermentation de MEL(s) décrit ci-avant, optionnellement suivi d'une étape de purification.
Avantageusement, le(s) ester(s) méthylique(s) ou éthylique(s) d'acide(s) gras et le(s) tensioactif(s) non-ionique(s) de HLB supérieur ou égal à 12 utilisés dans le procédé présentent les caractéristiques de ces composants telles qu'elles sont décrites ci-avant.
Selon un premier mode de réalisation du procédé de préparation d'une composition selon l'invention, celui-ci est un procédé de préparation d'une composition phytosanitaire et comporte une étape de mélangeage d'au moins un ester méthylique ou éthylique d'acide gras, d'au moins un lipide de mannosylérythritol, et d'au moins un tensioactif non-ionique de HLB supérieur ou égal à 12, avec un principe pesticide.
Selon un second mode de réalisation du procédé de préparation d'une composition selon l'invention, celui-ci est un procédé de préparation d'une composition cosmétique et comporte une étape de mélangeage d'au moins un ester méthylique ou éthylique d'acide gras, d'au moins un lipide de mannosylérythritol, et d'au moins un tensioactif non-ionique de HLB supérieur ou égal à 12, avec un principe actif cosmétique.
Avantageusement, le principe actif pesticide ou le principe actif cosmétique utilisé dans les deux modes de réalisation décrits ci-avant présentent les caractéristiques de ces composants telles qu'elles sont décrites ci-avant.
L'invention concerne en outre une émulsion comprenant une composition selon l'invention et de l'eau.
Les compositions selon l'invention peuvent en effet être utilisées en tant que concentré émulsionnable.
Lorsqu'une émulsion est préparée à partir de la composition selon l'invention, celle-ci est stable dans le temps. Par « stable », on entend que l'émulsion ne montre pas ou montre peu de séparation de phases après 30 secondes, de préférence après 30 minutes, plus préférentiellement après 2 heures, encore plus préférentiellement après 24 heures de stockage. Avantageusement, la stabilité d'une émulsion préparée à partir de la composition selon l'invention est caractérisée conformément à l'étape (ii) de la norme CIPAC MT 36.3.
Par ailleurs, une émulsion préparée à partir de la composition selon l'invention ne comprend pas ou comprend peu de mousse.
La stabilité d'émulsions préparées à partir de la composition selon l'invention est plus amplement décrite dans l'Exemple 3.
De plus, il a été mis en évidence par les inventeurs que, de façon surprenante, une émulsion comprenant la

composition selon l'invention a un pouvoir mouillant élevé. Ainsi, lorsqu'une émulsion comprenant la composition selon l'invention est appliquée sur une surface solide (telle qu'une surface plane hydrophobe), le mouillage de cette surface solide par cette émulsion est élevé (voir Exemples 4 et 10).

Dans le cadre de la présente demande :

- par « mouillage », on entend l'étalement d'un liquide sur un solide ;
- par « tension superficielle » d'un liquide, on entend la force exercée à l'interface entre ce liquide et un solide. Les termes « tension superficielle » et « tension de surface » sont synonymes dans la présente demande ;
- par angle de contact d'une goutte de liquide 1 déposée sur une surface solide plane 2, on entend l'angle θ formé par la tangente à la goutte de liquide 1 au point de contact avec la surface solide plane 2, tel que représenté à la Figure 1.

A titre d'exemple, lorsqu'un liquide, tel qu'une goutte de solution ou d'émulsion, et un solide, tel qu'une paroi ou une feuille végétale, sont mis en contact, la capacité du liquide à mouiller le solide, c'est-à-dire à s'étaler ou se répandre sur celui-ci, dépendra directement de la force exercée à l'interface entre liquide et solide, laquelle est généralement définie comme la tension superficielle. La tension superficielle représente donc la force permettant au liquide d'adhérer au solide, ou l'empêchant de se répandre sur celui-ci. Ainsi plus la tension superficielle est importante, moins le liquide sera capable de mouiller le solide en question.

Plusieurs cas de figures peuvent donc être représentés pour illustrer la notion de mouillage. Les Figures 2a à 2c représentent plus particulièrement trois cas de figures.

Ainsi, comme illustré sur la Figure 2a, lorsqu'une goutte de liquide 1 tombe sur une surface solide plane 2, celle-ci peut réaliser un mouillage total de cette surface 2, c'est-à-dire se répandre sur toute la surface de celle-ci, en formant un film d'angle de contact θ égal à 0 avec ladite surface 2. Alternativement, la goutte 1 peut réaliser un mouillage partiel de la surface 2 (Figure 2b), c'est-à-dire ne pas se répandre totalement sur cette dernière, en formant une goutte d'angle de contact θ compris entre 0 et 90° avec ladite surface 2. Enfin, la goutte 1 peut ne pas mouiller du tout la surface 2 (Figure 2c), c'est-à-dire ne pas se répandre sur celle-ci, en formant une goutte d'angle de contact θ supérieur à 90° avec ladite surface 2.

Par ailleurs, une émulsion comprenant la composition selon l'invention a un pouvoir pénétrant élevé. Cette caractéristique est plus particulièrement décrite dans l'Exemple 10.

Ainsi, une émulsion phytosanitaire préparée à partir d'une composition selon l'invention aura une efficacité, ou une efficacité améliorée, par rapport à une émulsion phytosanitaire ne comprenant pas une telle composition, comme cela est démontré dans l'Exemple 11.

En outre, une émulsion comprenant la composition selon l'invention présente l'avantage de pouvoir être pulvérisée sous forme de gouttelettes de diamètre avantageusement supérieur à 100$\mu$m, ce qui permet de réduire le phénomène de dérive lors de la pulvérisation.

En outre, une émulsion comprenant la composition selon l'invention a une couleur blanche particulièrement favorable à son utilisation dans divers domaines, comme par exemple en cosmétique. Cette caractéristique est plus particulièrement mise en évidence à la Figure 5.

L'eau est choisie en fonction de l'utilisation envisagée pour l'émulsion, ou de la nature de la composition à partir de laquelle l'émulsion est préparée.

Par exemple, dans le cadre de la préparation d'une émulsion à partir d'une composition phytosanitaire, l'eau est du type de celle utilisée dans le domaine phytosanitaire, telle qu'une eau de forage, qui peut être une eau moyenne à dure. Avantageusement, l'eau moyenne à dure présente une dureté comprise entre 300 et 600 ppm, préférentiellement entre 450 et 550 ppm. Une telle émulsion est généralement destinée à être pulvérisée, par exemple par un agriculteur sur des cultures.

Dans le cadre de la préparation d'une émulsion à partir d'une composition cosmétique, l'eau est une eau généralement utilisée en cosmétique, telle qu'une eau distillée ou une eau osmosée.

Avantageusement la quantité de composition selon l'invention dans l'émulsion selon l'invention est comprise entre 0,01 et 20% en poids, de préférence entre 0,05 et 10% en poids, plus préférentiellement encore entre 0,1 et 5% en poids, par rapport au poids total de l'émulsion.

De préférence, la quantité d'eau dans l'émulsion selon l'invention est comprise entre 50 et 99,99% en poids, de préférence entre 80 et 99,9% en poids, plus préférentiellement entre 85 et 99,9% en poids, par rapport au poids total de l'émulsion.

En particulier, la quantité d'eau peut être comprise entre 90 et 99,5% en poids, par rapport au poids total de l'émulsion.

Avantageusement, l'émulsion selon l'invention est une émulsion huile dans eau.

L'invention concerne également un procédé de préparation d'une émulsion selon l'invention, comprenant une étape de mélangeage d'une composition selon l'invention avec de l'eau.

Avantageusement, lors de l'étape de mélangeage, les composants nécessitent une faible agitation.
L'invention concerne en outre l'utilisation d'une composition selon l'invention en tant qu'adjuvant.
De préférence, l'adjuvant a une propriété mouillante et/ou pénétrante.
L'invention sera mieux comprise au vu des exemples qui suivent, donnés à titre illustratif, avec référence aux Figures suivantes :

- La Figure 1, qui représente l'angle de contact θ formé par la tangente à une goutte de liquide 1 au point de contact avec une surface solide plane 2 ;
- La Figure 2, qui représente trois cas de figures illustrant la notion de mouillage, à savoir le cas d'un mouillage total d'une surface solide plane 2 par une goutte de liquide 1 (Fig. 2a), le cas d'un mouillage partiel d'une surface solide plane 2 par une goutte de liquide 1 (Fig. 2b), le cas où une goutte de liquide 1 ne mouille pas une surface solide plane 2 (Fig. 2c) ;
- La Figure 3 qui est un diagramme représentant la diminution de l'angle de contact obtenue avec des émulsions comprenant une composition selon l'invention et des émulsions comprenant des compositions comparatives ;
- La Figure 4, qui est un diagramme représentant la diminution de l'angle de contact obtenue avec des émulsions comprenant différents tensioactifs non-ioniques de HLB supérieur ou égal à 12 ;
- La Figure 5, qui est une photographie d'une émulsion comprenant une composition selon l'invention (portant le numéro 1 sur la photographie) et d'une émulsion comprenant une composition comparative (portant le numéro 3 sur la photographie) ;
- La Figure 6, qui est un diagramme représentant l'évolution de la teneur en principe actif pesticide au cours du temps d'une composition phytosanitaire selon l'invention ;
- La Figure 7, qui est un diagramme représentant la diminution de l'angle de contact obtenue avec une émulsion préparée à partir d'une composition selon l'invention, et avec des émulsions comprenant des compositions comparatives ;
- La Figure 8, qui est un diagramme représentant l'intensité de la fluorescence émise par les cellules de plantes traitées avec un contrôle (eau), avec une émulsion préparée à partir d'une composition selon l'invention et avec une émulsion comprenant une composition comparative ;
- La Figure 9, qui comporte deux photographies représentant respectivement l'intensité de la fluorescence émise par les cellules de plantes traitées avec une émulsion préparée à partir d'une composition selon l'invention (photographie a) et avec une émulsion comprenant une composition comparative (photographie b) ;
- La Figure 10, qui comporte quatre photographies représentant respectivement l'intensité de la fluorescence (24 à 48h après traitement) émise par les cellules d'une racine de plante traitée avec une émulsion comprenant une composition selon l'invention et du glyphosate (photographie d), avec une solution contrôle (eau) (photographie a), avec une solution de glyphosate seul (photographie b) et avec une émulsion comprenant une composition selon l'invention seule (photographie c) ;
- La Figure 11, qui est un diagramme représentant l'efficacité d'un traitement de plantes avec une émulsion comprenant une composition selon l'invention et du glyphosate, avec une solution contrôle (eau), avec une solution de glyphosate seul et avec une émulsion comprenant une composition selon l'invention seule;
- La Figure 12, qui comporte quatre photographies représentant respectivement des plantes traitées avec une émulsion comprenant une composition selon l'invention et du glyphosate (photographie d), avec une solution contrôle (eau) (photographie a), avec une solution de glyphosate seul (photographie b) et avec émulsion comprenant une composition selon l'invention seule (photographie c).

## Exemple 1 : Préparation d'une composition selon l'invention

1. Obtention de MELs

Les MELs ont été obtenus par un procédé de fermentation comprenant les étapes suivantes :

- la culture d'une souche de levure telle que *Pseudozyma aphidis* en présence d'huile végétale (colza) pour obtenir les MELs; et
- la récupération des MELs ainsi obtenus.

A l'issue de l'étape de récupération des MELs, un mélange de MELs est obtenu, qui présente les caractéristiques suivantes :

- Teneur en MELs: 55% en poids
- Teneur en autres composants : 45% en poids (dont 42% en poids d'acides gras libres et de triglycérides et 3% en

poids d'eau et de souche),

les pourcentages en poids étant donnés par rapport au poids total du mélange de MELs obtenu.

En particulier, le mélange de MELs comporte des MELs-A à une teneur de 52% en poids, des MELs-B à une teneur de 12% en poids, des MELs-C à une teneur de 35% en poids, et des MELs-D à une teneur de 1% en poids, les pourcentages en poids étant donnés par rapport au poids de la quantité totale de MELs.

2. Esters méthyliques d'acides gras

Du Radia® 7955 de chez OLEON NV a été utilisé.

Le Radia® 7955 est composé d'esters méthyliques d'acides gras d'huile de colza (« EMC »).

3. Alcool

Du 2-octanol de chez Sigma-Aldrich a été utilisé.

4. Tensioactif non-ionique de HLB supérieur ou égal à 12

Du mono-oléate de polyéthylène glycol-600 (« PEG-600-oléate ») commercialisé par OLEON NV sous la marque RADIA® 7404 a été utilisé (HLB : 13,2).

5. Procédé de préparation de compositions selon l'invention

Composition 1

Dans un flacon en verre de 60 mL, 4% en poids du mélange de MELs, 93% en poids de Radia® 7955 et 3% en poids de Radia® 7404 ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation.

Composition 2

Dans un flacon en verre de 60 mL, 4% en poids du mélange de MELs, 90% en poids de Radia® 7955, 3% en poids de 2-octanol et 3% en poids de Radia® 7404 ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation.

## Exemple 2 : Evaluation de l'émulsionnabilité de compositions selon l'invention et de compositions comparatives

### 1. Matériels et méthodes

#### 1.1 Matériel

Les produits qui ont été utilisés dans cet Exemple sont les suivants :

- les compositions 1 à 2 selon l'invention préparées à l'Exemple 1
- Actirob B® (adjuvant phytosanitaire à base d'esters méthyliques d'acides gras d'huile de colza, comprenant un mélange de tensioactif(s) ionique(s) et non-ionique(s), commercialisé par OLEON NV)
- le mélange de MELs préparé à l'Exemple 1
- des esters méthyliques d'acides gras d'huile de colza (Radia® 7955, OLEON NV)
- du mono-oléate de polyéthylène glycol-600 (Radia® 7404, OLEON NV, HLB : 13,2)
- du 2-octanol (Sigma-Aldrich)
- de l'eau standard C (Préparée selon la norme CIPAC MT 18.1.3)

Le matériel suivant a également été utilisé dans cet Exemple :

- des flacons en verre de 60 mL
- des éprouvettes graduées de 100 mL

- des pipettes graduées de 5 mL

**1.2. Méthodes**

Compositions selon l'invention

Les compositions 1 et 2 préparées à l'Exemple 1 ont été utilisées.

Préparation des compositions comparatives

Composition 3 comparative

Cette composition comprend uniquement de l'Actirob B.

Composition 4 comparative

Dans un flacon en verre de 60 mL, 2% en poids du mélange de MELs et 98% en poids d'Actirob B® ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation.

Composition 5 comparative

Dans un flacon en verre de 60 mL, 94% en poids de Radia® 7955, 3% en poids de 2-octanol et 3% en poids de Radia® 7404 ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation.

Composition 6 comparative

Dans un flacon en verre de 60 mL, 97% en poids de Radia® 7955 et 3% en poids de Radia® 7404 ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation.

Composition 7 comparative

Dans un flacon en verre de 60 mL, 96% en poids de Radia® 7955 et 4% en poids du mélange de MELs ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation.

Evaluation de l'émulsionnabilité des compositions 1 à 7

Un protocole de préparation d'émulsions à partir des compositions 1 et 2 selon l'invention et des compositions 3 à 7 comparatives a été réalisé, conformément à l'étape (i) de la norme CIPAC MT 36.3 (« CIPAC method 2000. Prepared by the German Formulation Panel (DAPF). Chairman : G Menschel. »).

Dans des éprouvettes graduées de 100 mL, 1% en poids des différentes compositions 1 à 7 a respectivement été ajouté à 99% en poids d'eau standard. Les éprouvettes graduées ont été refermées à l'aide d'un bouchon. Les éprouvettes graduées ont ensuite été retournées une fois. Conformément à la note 4 de la norme CIPAC MT 36.3, lorsqu'une éprouvette est « retournée une fois », on entend que l'éprouvette est retournée manuellement de sorte à pivoter d'un angle de 180°C, puis ramenée à sa position initiale, le tout en environ deux secondes.

Après 30 secondes, il a été observé à l'œil nu si une émulsion s'est formée ou non dans chaque éprouvette. Lorsqu'une émulsion s'est formée, il a été observé si celle-ci était uniforme (complète), ou au contraire incomplète. Lorsqu'une émulsion uniforme se forme, cela signifie que la composition a une excellente émulsionnabilité. Au contraire, lorsque l'émulsion est incomplète, cela signifie que la composition n'a pas une bonne émulsionnabilité.

Les résultats sont présentés dans le Tableau 1 ci-dessous.

Tableau 1 : Emulsionnabilité des compositions 1 à 7

| Composition ajoutée à l'eau standard | Observation à 30 secondes |
|---|---|
| Composition 1 selon l'invention (4% mélange de MELs, 93% EMC, 3% PEG-600-oleate) | Emulsion uniforme |
| Composition 2 selon l'invention (4% mélange de MELs, 90% EMC, 3% 2-octanol, 3% PEG-600-oleate) | Emulsion uniforme |
| Composition 3 comparative (Actirob B®) | Emulsion uniforme |
| Composition 4 comparative (2% mélange de MELs, 98% Actirob B®) | Pas d'émulsion formée |
| Composition 5 comparative (94% EMC, 3% 2-octanol, 3% PEG-600-oleate) | Emulsion uniforme |
| Composition 6 comparative (97% EMC, 3% PEG-600-oleate) | Emulsion uniforme |
| Composition 7 comparative (96% EMC, 4% mélange de MELs) | Pas d'émulsion formée |

Les résultats présentés dans le Tableau 1 montrent que les compositions selon l'invention, lorsqu'elles sont ajoutées à de l'eau, permettent la formation d'émulsions uniformes, et ce de façon spontanée (après une faible agitation : un seul retournement manuel). Les compositions selon l'invention présentent donc une excellente émulsionnabilité.

Les émulsions 1 à 3, 5 et 6 préparées à partir des compositions 1 à 3, 5 et 6 ont été utilisées dans l'Exemple 3 ci-après.

**Exemple 3 : Evaluation de la stabilité d'émulsions préparées à partir de compositions selon l'invention et de compositions comparatives**

La stabilité des émulsions 1 à 3, 5 et 6 préparées dans l'Exemple 2 a été évaluée, conformément à l'étape (ii) de la norme CIPAC MT 36.3. Lorsqu'une émulsion est stable, celle-ci apparaît sous la forme d'une seule phase. Au contraire, lorsqu'une émulsion est instable, il peut être observé à l'œil nu une séparation de la phase huileuse et de la phase aqueuse. Cette séparation de phases se traduit par la présence de crème, qui représente la phase aqueuse en cours de séparation, et d'huile libre, qui représente la phase huileuse en cours de séparation. Le niveau de séparation de la phase huileuse et de la phase aqueuse peut être quantifié par les volumes de crème et d'huile libre présents dans l'émulsion. En outre, la préparation d'une émulsion peut résulter en la formation de mousse. A l'échelle industrielle, l'inconvénient de la formation de mousse lors de la préparation d'une émulsion phytosanitaire est que l'utilisateur ne pourra verser la quantité d'eau requise pour la préparation de cette émulsion, ce qui peut résulter en une trop forte concentration en principe actif phytosanitaire, ou même à un débordement de la cuve contenant l'émulsion. La quantité de mousse peut être quantifiée par le volume de mousse présent dans l'émulsion.

Evaluation de la stabilité des émulsions 1 à 3, 5 et 6

Suite à l'observation à 30 secondes des émulsions 1 à 3, 5 et 6 de l'Exemple 2, les éprouvettes contenant ces émulsions ont été retournées dix fois puis déposés dans une pièce où elles sont restées pendant 24 heures à une température constante de 20+/- 2°C. A 30 minutes, à 1 heure, à 2 heures et à 24 heures, les volumes d'huile libre et/ou de crème formée en haut ou en bas des émulsions, ont été mesurés par lecture du volume correspondant sur les éprouvettes graduées. Les volumes de mousse ont également été mesurés de la même manière.

Résultats

Les résultats sont présentés dans les Tableaux 2 et 3, ci-après.

Tableau 2 : Stabilité des émulsions 1 et 2 selon l'invention

| | Emulsion 1 | | | Emulsion 2 | | |
|---|---|---|---|---|---|---|
| | Mousse (mL) | Crème (mL) | Huile (mL) | Mousse (mL) | Crème (mL) | Huile (mL) |
| 30 minutes | 1 | 0 | 0 | 1 | 0 | 0 |
| 1 heure | 1 | 0 | 0 | 0,5 | 0 | 0 |

(suite)

| | Emulsion 1 | | | Emulsion 2 | | |
|---|---|---|---|---|---|---|
| | Mousse (mL) | Crème (mL) | Huile (mL) | Mousse (mL) | Crème (mL) | Huile (mL) |
| 2 heures | 0 | 1 | 0,2 | 0,5 | 1 | 0 |
| 24 heures | 0 | 0 | 1 | 0 | 1 | 0 |

Tableau 3 : Stabilité des émulsions 3, 5 et 6 comparatives

| | Emulsion 3 comparative | | | Emulsion 5 comparative | | | Emulsion 6 comparative | | |
|---|---|---|---|---|---|---|---|---|---|
| | Mousse (mL) | Crème (mL) | Huile (mL) | Mousse (mL) | Crème (mL) | Huile (mL) | Mousse (mL) | Crème (mL) | Huile (mL) |
| 30 minutes | 0 | 0,5 | 0 | 3 | 0,5 | 0 | 3 | 0,5 | 0 |
| 1 heure | 0 | 0,5 | 0 | 2 | 1 | 0 | 2 | 1 | 0 |
| 2 heures | 0 | 1 | 0 | 1 | 1 | 0 | 1 | 1 | 0 |
| 24 heures | 0 | 2 | 0 | 0 | 2 | 0 | 0 | 2 | 0 |

Les résultats présentés dans les Tableaux 2 et 3 montrent que les émulsions 1 et 2 selon l'invention présentent une meilleure stabilité que les émulsions 3, 5 et 6 comparatives. En particulier, l'apparition de crème dans les émulsions 1 et 2 selon l'invention est moins rapide que celle dans les émulsions 3, 5 et 6 comparatives.

Par ailleurs, les émulsions 1 et 2 selon l'invention comportent moins de mousse que les émulsions 3, 5 et 6 comparatives.

De plus, en comparant les émulsions 1 et 2, on note, après 24h, qu'il y a apparition d'huile en surface de l'émulsion 1 ne comprenant pas d'alcool. Ce phénomène n'est pas observable pour l'émulsion 2, qui est de composition identique à celle de l'émulsion 1 mais qui comprend également un alcool. Par conséquent, ce dernier permet de mieux stabiliser l'émulsion dans le temps.

En outre, une émulsion comprenant une composition selon l'invention a une couleur très blanche, ce qui la rend particulièrement adaptée à une utilisation cosmétique.

A titre d'exemple, des photographies de l'émulsion 1 comprenant la composition 1 selon l'invention (portant le numéro 1 sur la photographie) et de l'émulsion 6 comprenant la composition 6 comparative (portant le numéro 3 sur la photographie) sont présentées à la Figure 5. Ces photographies ont été prises 5 minutes après avoir retourné dix fois les éprouvettes contenant ces émulsions.

**Exemple 4 : Evaluation du pouvoir mouillant d'émulsions préparées à partir de compositions selon l'invention et de compositions comparatives**

1. Matériels et Méthodes

1.1. Matériel

Les émulsions 1 à 2 selon l'invention, et les émulsions 3, 5 et 6 comparatives préparées dans l'Exemple 2 ont été utilisées. Une solution contrôle d'eau standard a également été préparée.

Le matériel suivant a été utilisé:

- des flacons en verre,
- une seringue de 1 mL munie d'une aiguille de diamètre 0,63 mm
- un parafilm hydrophobe (Parafilm « M », NEENAH, WI 54956)
- le goniomètre DSA10 (KRUSS)
- le logiciel « Drop shape analysis » (KRUSS)

2. Méthodes

Mesure des angles de contact

Des mesures d'angle de contact ont été réalisées pour chacune des émulsions 1,2, 3, 5, 6 et de la solution contrôle, à l'aide du goniomètre.

Pour cela, une goutte de chaque émulsion et de la solution contrôle (3µL) a été formée à l'aide de la seringue. La seringue a ensuite été placée à environ 0,5 cm au-dessus du parafilm hydrophobe. Par la pesanteur, cette goutte s'est détachée de l'aiguille et est tombée sur le parafilm hydrophobe. Le suivi de la variation de l'angle de contact a été assuré dès le moment où la goutte a touché le parafilm, et ce pendant 10 minutes, grâce au logiciel d'analyse.

Les résultats ont ensuite été traités afin de comparer les variations d'angles de contact pour chacune des émulsions 1 à 3, 5 et 6 par rapport à la solution contrôle.

**2. Résultats**

Les résultats des mesures d'angle de contact pour chacune des émulsions 1 à 3, 5 et 6 et de la solution contrôle sont présentés à la Figure 3.

Les résultats montrent que la diminution de l'angle de contact obtenue avec les émulsions 1 et 2 selon l'invention est supérieure à celle obtenue avec chacune des émulsions 3, 5 et 6 comparatives. Les émulsions 1 et 2, comprenant la composition selon l'invention, ont donc un pouvoir mouillant supérieur à celui des émulsions 3, 5 et 6, comprenant des compositions comparatives.

**Exemple 5 : Effet de différents tensioactifs non-ioniques de HLB supérieur ou égal à 12 sur le pouvoir mouillant d'émulsions les comprenant**

3. Matériels et Méthodes

3.1. Matériel

Les produits suivants ont été utilisés :

- le mélange de MELs préparé à l'Exemple 1
- des esters méthyliques d'acides gras d'huile de colza (Radia® 7955, OLEON NV)
- du mono-oléate de polyéthylène glycol-600 (Radia® 7404, OLEON NV)
- du polysorbate 20 (Radia® 7137, OLEON NV, HLB : 16,5)
- du polysorbate 80 (Radia® 7157, OLEON NV, HLB : 14,9)
- de l'eau standard C

Le matériel suivant a été utilisé:

- des flacons en verre,
- une seringue de 1 mL munie d'une aiguille de diamètre 0,63 mm
- un parafilm hydrophobe (Parafilm « M », NEENAH, WI 54956)
- le goniomètre DSA10 (KRUSS)
- le logiciel « Drop shape analysis » (KRUSS)

4. Méthodes

Préparations des compositions

La composition 1 selon l'invention a été utilisée.

Composition 8 selon l'invention

Dans un flacon en verre de 60 mL, 4% en poids du mélange de MELs, 93% en poids de Radia® 7955, et 3% en poids de polysorbate 20 ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation.

Composition 9 selon l'invention

Dans un flacon en verre de 60 mL, 4% en poids du mélange de MELs, 93% en poids de Radia® 7955, et 3% en poids de polysorbate 80 ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation.

Préparations des émulsions

Des émulsions 1, 8 et 9 comprenant respectivement les compositions 1 (émulsion 1), 8 (émulsion 8) et 9 (émulsion 9), ont été préparées conformément à l'étape (i) de la norme CIPAC MT 36.3, comme indiqué ci-après.
Dans des flacons, 1% en poids de chacune des compositions 1, 8 et 9 a respectivement été ajouté à 99% en poids d'eau standard. Les flacons ont été refermés à l'aide d'un bouchon. Les flacons ont ensuite été retournés une fois pour obtenir les émulsions 1, 8 et 9.
Une solution contrôle d'eau standard a également été préparée.

Mesure des angles de contact

Des mesures d'angle de contact ont été réalisées pour chacune des émulsions 1, 8, 9 et de la solution contrôle, à l'aide du goniomètre.
Pour cela, une goutte de chaque émulsion ou de la solution contrôle (3μL) a été formée à l'aide de la seringue. La seringue a ensuite été placée à environ 0,5 cm au-dessus du parafilm hydrophobe. Par la pesanteur, cette goutte s'est détachée de l'aiguille et est tombée sur le parafilm hydrophobe. Le suivi de la variation de l'angle de contact a été assuré dès le moment où la goutte a touché le parafilm, et ce pendant 10 minutes, grâce au logiciel d'analyse.
Les résultats ont ensuite été traités afin de comparer les variations d'angles de contact pour chacune des émulsions 1, 8 et 9, par rapport à la solution contrôle.

**2. Résultats**

Les résultats des mesures d'angle de contact pour chacune des émulsions 1, 8, 9 et de la solution contrôle sont présentés à la Figure 4.
Les résultats montrent que la diminution de l'angle de contact obtenue avec l'émulsion 1 est supérieure à celle obtenue avec chacune des émulsions 8 et 9.

**Exemple 6 : Utilisation d'une composition phytosanitaire selon l'invention dans la préparation d'une émulsion**

Une composition phytosanitaire selon l'invention a été préparée puis utilisée dans la préparation d'une émulsion.
La composition phytosanitaire selon l'invention présente les caractéristiques suivantes :

| Composants | % en poids* |
|---|---|
| Mélange de MELs | 4% |
| Radia® 7955 | 79,95% |
| Radia® 7404 | 3% |
| 2-octanol | 3% |
| Cinnamaldéhyde (Herbarom laboratoire) | 10% |
| Eugénol (Sigma-Aldrich) | 0,05% |
| *Pourcentage en poids par rapport au poids total de la composition phytosanitaire. | |

L'émulsion a été préparée comme suit :
Dans un récipient refermable, 50% en poids d'eau, 1% en poids de la composition phytosanitaire selon l'invention, puis 49% en poids d'eau ont successivement été ajoutés, les pourcentages en poids étant indiqués par rapport au poids total du mélange composition phytosanitaire-eau obtenu. Le récipient a ensuite été refermé puis retourné pour obtenir l'émulsion. Si nécessaire, le pH et la salinité de l'eau auront été ajusté au préalable.
L'émulsion selon l'invention obtenue présente les caractéristiques suivantes :

| Composants | % en poids* |
|---|---|
| Composition phytosanitaire selon l'invention | 1% |
| Eau | 99% |
| *Pourcentage en poids par rapport au poids total de l'émulsion. | |

**Exemple 7 : Evaluation de la teneur en principe actif pesticide au cours du temps dans une composition phytosanitaire selon l'invention**

L'évolution de la teneur en cinnamaldéhyde au cours du temps de la composition phytosanitaire selon l'invention préparée dans l'Exemple 6 a été analysée par chromatographie en phase gazeuse (analyse GC).

1. Matériel

Le matériel suivant a été utilisé :

- une balance de précision à 0,00001 g
- des pipettes automatiques de 1 et 5 mL
- des pipettes Pasteur
- des fioles jaugées de 20 et 100 mL
- un chromatographe pour chromatographie en phase gazeuse (GC 6580 - Agilent Technologies)
- une seringue de 5μL

2. Méthodes

La composition phytosanitaire selon l'invention préparée dans l'Exemple 6 a été stockée pendant deux mois au cours desquels une analyse GC a été réalisée à différents temps (fabrication, 0 jours, 15 jours, 1 mois, 2 mois, 3 mois).
Le stockage a été réalisé :

- à température ambiante (environ 20°C en conditions normales de température et de pression (CNTP)), ou
- à 54°C.

Les paramètres de l'analyse GC sont les suivants :

- injecteur : split mode ; température : 350°C ; split ratio : 10 :1
- phase mobile : Hélium
- débit gaz vecteur : 2 mL/min
- temps d'analyse : 36 minutes
- volume d'injection : 2μL
- détecteur : FID ; température : 250°C ; air : 450 mL/min ; H2 : 40 mL/min ; He : 30 mL/min
- colonne : DB5 HT 15m*250μm*0,1μm

Tous les composants utilisés dans la préparation de la composition phytosanitaire selon l'invention ont été passés seuls en GC selon les paramètres GC ci-dessus. Ceci a permis de vérifier qu'aucun de ces composants n'avait un temps de rétention identique au cinnamaldéhyde ou à l'étalon interne (azulène).
Plus particulièrement :

- le temps de rétention du cinnamaldéhyde est de 6 min.
- le temps de rétention de l'azulène est de 6,7 min.
- les composants ont des temps de rétention différents de ceux du cinnamaldéhyde et de l'azulène.

La sélectivité de la méthode est conforme.
Lors d'une 1$^{ère}$ étape, l'étalonnage interne avec des solutions de cinnamaldéhyde à différentes concentrations a été réalisé.

Ensuite, la $2^{ème}$ étape consistant en l'analyse GC a été réalisée, suivi du calcul du taux de cinnamaldéhyde dans la composition phytosanitaire, à l'aide de la courbe étalon.

Les résultats de l'analyse GC sont présentés dans le Tableau 4 ci-dessous et à la Figure 6.

Tableau 4 : Résultats de l'analyse GC de la composition phytosanitaire selon l'invention

| Temps | Teneur en cinnamaldéhyde | | Limites min 10% | Limites max 10% |
|---|---|---|---|---|
| | Température ambiante | 54 °C | | |
| Fabrication | 10 | 10 | 9 | 11 |
| 0 jours | 9,71 | 9,71 | 9 | 11 |
| 15 jours | 9,91 | 10,07 | 9 | 11 |
| 1 mois | 9,89 | 9,88 | 9 | 11 |
| 2 mois | 9,74 | 9,57 | 9 | 11 |
| 3 mois | 10,22 | 9,81 | 9 | 11 |

Les résultats montrent que la teneur en cinnamaldéhyde de la composition phytosanitaire selon l'invention diminue très faiblement au cours du temps, et ce quelles que soient les conditions de stockage.

**Exemple 8 : Utilisation d'une composition cosmétique selon l'invention dans la préparation d'une émulsion**

Une composition cosmétique selon l'invention a été préparée puis utilisée dans la préparation d'une émulsion.

La composition cosmétique selon l'invention présente les caractéristiques suivantes :

| Composants | % en poids* |
|---|---|
| Mélange de MEL(s) | 2% |
| Methyl olivate (PEL-EST® OME - Elé corporation) | 93% |
| Radia® 7404 | 1,5% |
| Alcool laurique | 1,5% |
| Huile de jojoba | 2% |
| *Pourcentage en poids par rapport au poids total de la composition cosmétique. | |

L'émulsion a été préparée comme suit :
Dans un flacon, 50% en poids d'eau, 5% en poids de la composition selon l'invention, et 45% en poids d'eau ont successivement été ajoutés, les pourcentages en poids étant indiqués par rapport au poids total du mélange composition phytosanitaire-eau obtenu. Le flacon a ensuite été retourné pour obtenir l'émulsion. Si nécessaire, le pH et la salinité de l'eau auront été ajusté au préalable.

L'émulsion selon l'invention obtenue présente les caractéristiques suivantes :

| Composants | % en poids* |
|---|---|
| Composition cosmétique selon l'invention | 5% |
| Eau | 95% |
| *Pourcentage en poids par rapport au poids total de l'émulsion. | |

D'autres composants peuvent être ajoutés à cette émulsion, tels que d'autres principes actifs cosmétiques et/ou des agents de formulation, ces derniers permettant notamment de lui conférer les propriétés de texture (crème, gel) et/ou sensorielles souhaitées.

**Exemple 9 : Evaluation de la stabilité et du pouvoir mouillant d'émulsions préparées à partir d'une composition selon l'invention et de compositions comparatives**

1. Matériels et Méthodes

1.1. Matériel

Les produits suivants ont été utilisés :

- La composition 2 selon l'invention préparée à l'Exemple 1
- du polyglycérol-3 caprylate/caprate (PG-3-C8/C10, C094, OLEON)
- du Simulsol® SL11W (SEPPIC)
- des esters méthyliques d'acides gras d'huile de colza (« EMC », Radia® 7955, OLEON NV)
- du 2-octanol (Sigma-Aldrich)
- du mono-oléate de polyéthylène glycol-600 (Radia® 7404, OLEON)
- de l'eau standard D (préparée selon la norme CIPAC MT 18.1.4)

Le matériel suivant a été utilisé:

- des flacons en verre,
- des éprouvettes graduées,
- des pipettes graduées
- une seringue de 1 mL munie d'une aiguille de diamètre 0,63 mm
- un parafilm hydrophobe (Parafilm « M », NEENAH, WI 54956)
- le goniomètre DSA10 (KRUSS)
- le logiciel « Drop shape analysis » (KRUSS)

1.2. Méthodes

Préparation de la composition selon l'invention

La composition 2 de l'Exemple 1 a été utilisée.

Préparation des compositions comparatives 10 et 11

Composition comparative 10 :

Dans un flacon en verre de 120 mL, 4% en poids de polyglycérol-3 caprylate/caprate, 90% en poids de Radia® 7955, 3% en poids de 2-octanol et 3% en poids de Radia® 7404 ont été ajoutés, les % en poids étant indiqués par rapport au poids total de la composition obtenue, puis agités manuellement jusqu'à homogénéisation de la composition. Lors de l'agitation, la composition est chauffée à approximativement 50 °C pour faciliter l'homogénéisation.

Composition comparative 11:

Dans un flacon en verre de 120 mL, 4% en poids de Simulsol® SL11W, 90% en poids de Radia® 7955, 3% en poids de 2-octanol et 3% en poids de Radia® 7404 ont été ajoutés, les % en poids étant indiqués par rapport au poids total de la composition obtenue, puis agités manuellement jusqu'à homogénéisation de la composition. Lors de l'agitation, la composition est chauffée à approximativement 50°C pour faciliter l'homogénéisation.

Il est observé que les compositions comparatives 10 et 11 sont troubles à température ambiante, ainsi qu'après chauffage à 50°C. De plus, pour ces deux compositions, un dépôt est observé au fond du flacon 24 heures après agitation. Au contraire, la composition 2 selon l'invention est homogène à température ambiante, ainsi qu'après chauffage à 50°C. Aucun dépôt n'est observé au fond du flacon 24 heures après agitation.

Préparation d'émulsions à partir des compositions 2, 10 et 11

Un protocole de préparation d'émulsions à partir de la composition 2 selon l'invention et des compositions 10 et 11 comparatives a été réalisé, conformément à l'étape (i) de la norme CIPAC MT 36.3 (« CIPAC method 2000. Prepared by the German Formulation Panel (DAPF). Chairman : G Menschel. »).

Dans des éprouvettes graduées, 1% en poids des différentes compositions 2, 10 et 11 ont respectivement été ajoutées à 99% en poids d'eau standard, les % en poids étant indiqués par rapport au poids total du mélange eau-composition. Les flacons ont été refermés à l'aide d'un bouchon. Les flacons ont ensuite été retournés une fois.
Après 30 secondes, il a pu être observé à l'œil nu qu'une émulsion uniforme s'est formée à partir de la composition 2 comparative. Des émulsions non-uniformes (incomplètes) se sont formées à partir des compositions comparatives 10 et 11.

Evaluation de la stabilité des émulsions 2, 10 et 11

La stabilité des émulsions 2, 10 et 11 préparées a été évaluée, conformément à l'étape (ii) de la norme CIPAC MT 36.3.
Suite à l'observation à 30 secondes des émulsions 2, 10 et 11, les éprouvettes contenant ces émulsions ont été retournées dix fois puis déposées dans une pièce où elles sont restées pendant 24 heures à une température constante de 20+/- 2°C. A 30 minutes, à 1 heure, à 2 heures et à 24 heures, les volumes d'huile libre et/ou de crème formée en haut ou en bas des émulsions, ont été mesurés par lecture du volume correspondant sur les éprouvettes graduées. Les volumes de mousse ont également été mesurés de la même manière.

Résultats

Les résultats sont présentés dans le Tableau 5 ci-après.

Tableau 5 : Stabilité des émulsions 2, 10 et 11

| | Emulsion 2 | | | Emulsion 10 | | | Emulsion 11 | | |
|---|---|---|---|---|---|---|---|---|---|
| | Mousse (mL) | Crème (mL) | Huile (mL) | Mousse (mL) | Crème (mL) | Huile (mL) | Mousse (mL) | Crème (mL) | Huile (mL) |
| 30 minutes | 1 | 0 | 0 | 3 | 1 | 0 | 2 | 0,5 | 0,5 |
| 1 heure | 1 | 0 | 0 | 1 | 1 | 0 | 1 | 1 | 1 |
| 2 heures | 0 | 1 | 0,2 | 1 | 1 | 0 | 1 | 1 | 1 |
| 24 heures | 0 | 0 | 1 | 0 | 2 | 0 | 0 | 2 | 2 |

Les résultats présentés dans le Tableau 5 montrent que l'émulsion 2 selon l'invention présente une meilleure stabilité que les émulsions 10 et 11 comparatives. En particulier, l'apparition de crème dans l'émulsion 2 selon l'invention est moins rapide que celle dans les émulsions 10 et 11 comparatives, et il peut être observé que la crème n'est plus présente dans l'émulsion 2 à 24 heures.

2. Evaluation du pouvoir mouillant des émulsions 2, 10 et 11

Mesure des angles de contact

Des mesures d'angle de contact ont été réalisées pour chacune des émulsions 2, 10 et 11 et pour une solution contrôle d'eau standard D, à l'aide du goniomètre.
Pour cela, une goutte de chaque émulsion ou de la solution contrôle (3μL) a été formée à l'aide de la seringue. La seringue a ensuite été placée à environ 0,5 cm au-dessus du parafilm hydrophobe. Par la pesanteur, cette goutte s'est détachée de l'aiguille et est tombée sur le parafilm hydrophobe. Le suivi de la variation de l'angle de contact a été assuré dès le moment où la goutte a touché le parafilm, et ce pendant 10 minutes, grâce au logiciel d'analyse.
Les résultats ont ensuite été traités afin de comparer les variations d'angles de contact pour chacune des émulsions 2, 10 et 11, par rapport à la solution contrôle.

Résultats

Les résultats des mesures d'angle de contact pour chacune des émulsions 2, 10 et 11 et de la solution contrôle sont présentés à la Figure 7.

Les résultats montrent notamment que la diminution de l'angle de contact obtenue avec l'émulsion 2 est notablement supérieure à celle obtenue avec chacune des émulsions 10 et 11.

**Exemple 10 : Evaluation du pouvoir pénétrant et du pouvoir mouillant d'émulsions préparées à partir d'une composition selon l'invention et à partir d'une composition comparative**

Cet exemple repose sur l'utilisation d'un fluorochrome émettant une fluorescence verte lors de son entrée dans les cellules végétales. Ainsi, une visualisation directe du pouvoir pénétrant et du pouvoir mouillant d'un produit à tester peut être réalisée à l'aide de ce fluorochrome. La quantification de l'intensité de la fluorescence est effectuée par analyse d'image à l'aide d'un logiciel adapté. Ceci permet de quantifier le taux de pénétration du fluorochrome.

1. Matériels et Méthodes

1.1. Matériel

Les produits suivants ont été utilisés :

- la composition 2 selon l'invention préparée à l'Exemple 1
- la composition 3 comparative préparée à l'Exemple 2
- de l'eau
- un fluorochrome
- des plants de chénopodes

1.2. Méthodes.

Les compositions 2 et 3 ont été préparées 15 minutes avant le traitement des chénopodes, pour assurer une bonne homogénéité de celles-ci.
La composition 3 comprend uniquement de l'Actirob B®, qui est un adjuvant connu pour ses propriétés mouillante et pénétrante.
Une solution contrôle comprenant de l'eau uniquement a également été préparée.

Préparation d'émulsions 2' et 3' à partir des compositions 2 et 3

Des émulsions 2' et 3' ont été préparées en mélangeant 0,25% en poids de la composition 2 ou 3 à 99,75% en poids d'eau standard, les % en poids étant indiqués par rapport au poids total du mélange eau-composition.
Le fluorochrome a été ajouté à chacune des émulsions 2' et 3' et à la solution contrôle.
Le fluorochrome a été ajouté 2 minutes avant le traitement des chénopodes.

Evaluation de la fluorescence

Les émulsions 2' et 3' et la solution contrôle ont respectivement été déposées sur des chénopodes, et la fluorescence a été évaluée.
L'intensité de la fluorescence est représentative du pouvoir pénétrant des émulsions.
La taille de la surface sur laquelle est visible la fluorescence est représentative du pouvoir mouillant des émulsions.
Les résultats sont présentés aux Figures 8 et 9.
Sur ces Figures 8 et 9, on constate que l'intensité de la fluorescence émise par les cellules des plantes traitées avec l'émulsion 2' est élevée, ce qui témoigne du pouvoir pénétrant de l'émulsion 2' comprenant une composition selon l'invention.
En outre, il peut être observé sur la Figure 9 que la fluorescence est émise sur une plus grande surface par les cellules de plantes traitées avec l'émulsion comprenant la composition 2 selon l'invention (Figure 9a) que par les cellules de plantes traitées avec l'émulsion comprenant la composition 3 comparative (Figure 9b). Le pouvoir mouillant de l'émulsion préparée à partir de la composition 2 selon l'invention est donc supérieur à celui de l'émulsion préparée à partir de la composition 3 comparative.

**Exemple 11 : Composition selon l'invention en combinaison avec un principe actif pesticide**

La composition 2 selon l'invention préparée à l'Exemple 1 a été utilisée en combinaison avec un principe actif

herbicide (le glyphosate) dans le traitement de chénopodes (*Chenopodium album*). Les plants de chénopodes utilisés étaient à un stade 2 à 3 feuilles (BBCH 12-13), et ont été cultivés dans un phytotron à une température de 15°C la nuit et 20°C le jour, avec 14 heures de photopériode. Le glyphosate utilisé était non formulé (ingrédient technique, référence 4521, Sigma-Aldrich®). Le glyphosate est connu pour être peu ou pas efficace lorsqu'il est utilisé seul car il n'adhère pas aux feuilles et pénètre difficilement.

Quatre traitements ont été effectués :

- un traitement contrôle (témoin), lors duquel 500g/ha d'eau supplémentée de 2% en poids d'agent fertilisant est pulvérisée sur les plants,
- un traitement au glyphosate seul, lors duquel 500 g/ha d'une solution à 0,25% en poids de glyphosate supplémentée de 2% en poids d'agent fertilisant est pulvérisée sur les plants,
- un traitement avec la composition 2 selon l'invention, lors duquel 500 g/ha d'une émulsion à 0,25% en poids de composition 2 selon l'invention et supplémentée de 2% en poids d'agent fertilisant est pulvérisée sur les plants,
- un traitement avec du glyphosate et la composition 2 selon l'invention, lors duquel une émulsion à 0,25% en poids de glyphosate et 0,25% en poids de composition 2 selon l'invention et supplémentée de 2% en poids d'agent fertilisant est pulvérisée sur les plants.

Les traitements ont été réalisés au moyen d'une buse Teejet® XR1100015 à 200l/ha.

Chaque traitement est réalisé sur 4 pots différents, chaque pot contenant 4 plants.

Les différents traitements réalisés sont présentés dans le Tableau 6 ci-dessous.

Tableau 6 : Traitements réalisés dans l'Exemple 11.

| Traitement | dose | Sulfate d'ammonium (agent fertilisant) |
|---|---|---|
| Solution contrôle (eau) | 500g/ha | 2% |
| Solution à 0,25% de glyphosate | 500g/ha | 2% |
| Emulsion à 0,25% de composition 2 selon l'invention | 500g/ha | 2% |
| Emulsion à 0,25% de glyphosate et 0,25% de composition 2 selon l'invention | 500g/ha | 2% |
| Les % sont des % en poids sur le poids total de solution ou d'émulsion. | | |

L'évaluation de l'efficacité a été réalisée par mesure de l'activité cellulaire des racines des plantes 24 à 48h après traitement. L'activité cellulaire est mesurée par coloration des cellules à l'aide de marqueurs fluorescents permettant de distinguer :

- les cellules ayant une activité métabolique (cellules vivantes), qui émettent une fluorescence de couleur verte ;
- les structures en fluorescence de couleur bleue ; et
- les cellules mortes, qui émettent une fluorescence de couleur rouge.

Lorsque l'activité métabolique cellulaire diminue, la fluorescence de couleur verte diminue. Les résultats sont présentés à la Figure 10.

Il peut être constaté une diminution de l'intensité de la fluorescence dans les cellules de racines de plantes traitées avec l'émulsion à 0,25% en poids de glyphosate et 0,25% en poids de composition 2 selon l'invention (Figure 10d). Cette diminution n'est pas observée pour les autres traitements avec la solution contrôle (Figure 10a), la solution à 0,25% en poids de glyphosate seul (Figure 10b) et l'émulsion à 0,25% en poids de composition 2 selon l'invention (Figure 10c).

L'évaluation de l'efficacité a également été réalisée par observation à l'œil nu des plantes 14 jours après traitement. L'efficacité est évaluée en pourcentage de destruction de la végétation, en prenant en compte la taille des plantes et les symptômes de phytotoxicité tels que la déformation des feuilles, l'apparition de taches blanches et la

nécrose.

Les résultats sont présentés dans le Tableau 7 ci-dessous et aux Figures 11 et 12.

Tableau 7 : Efficacité moyenne des traitements réalisés dans l'Exemple 11.

| Traitement | dose | Sulfate d'ammonium (agent fertilisant) | Efficacité (moyenne*) |
|---|---|---|---|
| Solution contrôle (eau) | 500g/ha | 2% | 0 |
| Solution de glyphosate | 500g/ha | 2% | 0 |
| Emulsion à 0,25% de composition 2 selon l'invention | 500g/ha | 2% | 0 |
| Emulsion à 0,25% de glyphosate et 0,25% de composition 2 selon l'invention | 500g/ha | 2% | 51,9 + /- 9,2 |
| Les % sont des % en poids sur le poids total de solution ou d'émulsion.<br>*Les résultats représentent l'efficacité moyenne pour chaque traitement, calculée sur la base des résultats obtenus pour les 4 pots différents comprenant les 4 plants de chénopodes. | | | |

Les résultats du Tableau 7 et des Figures 11 et 12 montrent que la phytotoxicité de l'émulsion à 0,25% en poids de glyphosate et 0,25% en poids de composition 2 selon l'invention est élevée (Figure 11 et Figure 12d). Cette phytotoxicité n'est pas observée pour les autres traitements avec la solution contrôle (Figure 11 et Figure 12a), la solution à 0,25% en poids de glyphosate seul (Figure 11 et Figure 12b) et l'émulsion à 0,25% en poids de composition 2 selon l'invention (Figure 11 et Figure 12c).

Ainsi, une composition selon l'invention peut être utilisée en combinaison avec un principe actif pesticide.

## Exemple 12 : Evaluation de l'émulsionnabilité de compositions comparatives comprenant un tensioactif non-ionique de HLB inférieur à 12

### 1. Matériels et méthodes

#### 1.1 Matériel

Les produits qui ont été utilisés dans cet Exemple sont les suivants :

- le mélange de MELs préparé à l'Exemple 1
- des esters méthyliques d'acides gras d'huile de colza (Radia® 7955 OLEON NV)
- du di-oléate de polyéthylène glycol-600 (Radia® 7444, OLEON NV, HLB : 10)
- du mono-oléate de polyéthylène glycol-200 (Radia® 7402, HLB : 7)
- du 2-octanol (Sigma-Aldrich)
- de l'eau standard D (préparée selon la norme CIPAC MT 18.1.4)

Le matériel suivant a également été utilisé dans cet Exemple :

- des flacons en verre de 60 mL
- des éprouvettes graduées de 100 mL
- des pipettes graduées de 5 mL

#### 1.2. Méthodes

Préparation des compositions comparatives

Composition 12 comparative

Dans un flacon en verre de 60 mL, 4% en poids du mélange de MELs, 90% en poids de Radia® 7955, 3% en poids de 2-octanol et 3% en poids de Radia® 7444 ont été ajoutés, les % en poids étant indiqués par rapport au poids total de la composition obtenue, puis agités manuellement jusqu'à homogénéisation de la composition. Lors de l'agitation,

il est possible de chauffer la composition à 40°C pour faciliter l'homogénéisation.

Composition 13 comparative

Dans un flacon en verre de 60 mL, 4% en poids du mélange de MELs, 90% en poids de Radia® 7955, 3% en poids de 2-octanol et 3% en poids de Radia® 7402 ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation.

Evaluation de l'émulsionnabilité des compositions comparatives 12 et 13

Un protocole de préparation d'émulsions à partir des compositions comparatives 12 et 13 a été réalisé, conformément à l'étape (i) de la norme CIPAC MT 36.3 (« CIPAC method 2000. Prepared by the German Formulation Panel (DAPF). Chairman : G Menschel. »).
Dans des éprouvettes graduées de 100 mL, 1% en poids des différentes compositions 12 et 13 a respectivement été ajouté à 99% en poids d'eau standard. Les éprouvettes graduées ont été refermées à l'aide d'un bouchon. Les éprouvettes graduées ont ensuite été retournées une fois.
Après 30 secondes, il a pu être observé à l'œil nu que des émulsions non-uniformes (incomplètes) se sont formées dans les éprouvettes : des gouttelettes étaient présentes dans chaque éprouvette.

**Exemple 13 : Composition selon l'invention en combinaison avec des principes actifs de biocontrôle**

La composition 2 selon l'invention préparée à l'Exemple 1 a été utilisée en combinaison avec des principes actifs de biocontrôle : la bactérie *Bacillus subtilis* et des spores du champignon *Gliocladium catenulatum.*
Des tests *in vitro* ont été réalisés sur plaques de micro-titration.
Des solutions aqueuses à différents pourcentages en poids (0%, 0,01%, 0,05%, 0,1%, 0,25%, 0,5%, 1%, 1,5%) de composition 2 selon l'invention ont été préparées.
*Bacillus subtilis* (souche QST 713) a été cultivée sur un milieu LPGA, et *Gliocladium catenulatum* (souche J1446) a été cultivé sur Malt-antibiotique.
Les suspensions de bactéries ou de spores obtenues ont ensuite respectivement été mises en contact avec chacune des solutions aqueuses préparées. Pour chaque solution aqueuse préparée, le test a été réalisé en triple, et des contrôles ont été effectués. Les contrôles consistaient en la mise en contact des suspensions de bactéries ou de spores avec une solution aqueuse pure. Les microplaques ont été incubées à 25°C pour *Bacillus subtilis,* et à 19 °C pour *Gliocladium catenulatum.*
La croissance des deux microorganismes a été évaluée par mesure de la densité optique (DO) à 590 nm afin de déterminer la $CI_{50}$ (concentration inhibitrice médiane) et la CMI (Concentration Minimale Inhibitrice) pour chaque micro-organisme.
Ces paramètres ont été mesurés 55 heures après la mise en contact avec les solutions pour *Bacillus subtilis,* et 146 heures après la mise en contact avec les solutions pour *Gliocladium catenulatum.*
Les résultats sont présentés ci-après :

*Bacillus subtilis* : $CI_{50}$ et CMI >1,5%
*Gliocladium catenulatum* : $CI_{50}$ et CMI >1,5%.

Ces résultats montrent que la composition 2 selon l'invention n'a aucun effet bactériostatique ou biotoxique sur *Bacillus subtilis* et *Gliocladium catenulatum.* La composition selon l'invention est donc compatible avec ces principes actifs de biocontrôle.

## Revendications

**1.** Composition comprenant :

- entre 75 et 99% en poids d'au moins un ester méthylique ou éthylique d'acide gras,
- entre 0,01 et 20% en poids d'au moins un lipide de mannosylérythritol, et
- entre 0,01 et 20% en poids d'au moins un tensioactif non-ionique de HLB supérieur ou égal à 12,

les pourcentages en poids étant indiqués par rapports au poids total de la composition.

2. Composition selon la revendication 1, comprenant entre 0,1 et 20% en poids d'au moins un alcool ayant un nombre d'atomes de carbone compris entre 1 et 16, par rapport au poids total de la composition.

3. Composition selon l'une quelconque des revendications 1 ou 2, dans laquelle la quantité totale de tensioactif(s) non-ionique(s) de HLB supérieur ou égal à 12 est comprise entre 0,5 et 10% en poids, par rapport au poids total de la composition.

4. Composition selon l'une quelconque des revendications 1 à 3, dans laquelle la quantité totale de MEL(s) est comprise entre 0,1 et 10% en poids, par rapport au poids total de la composition.

5. Composition selon l'une quelconque des revendications 1 à 4, comprenant au moins deux MELs choisis parmi le groupe constitué par MEL-A, MEL-B, MEL-C et MEL-D.

6. Composition selon l'une quelconque des revendications 1 ou 5, dans laquelle l'ester méthylique ou éthylique d'acide gras est un ester méthylique ou éthylique d'acide gras d'huile de colza, de soja et/ou d'olive.

7. Composition selon l'une quelconque des revendications 1 à 6, dans laquelle le tensioactif non-ionique de HLB supérieur ou égal à 12 est un ester d'acide gras et de polyéthylène glycol.

8. Composition selon l'une quelconque des revendications 1 à 7, comprenant en outre au moins un acide gras libre et/ou au moins un triglycéride.

9. Composition selon l'une quelconque des revendications 1 à 8, comprenant un principe actif pesticide.

10. Composition selon l'une quelconque des revendications 1 à 8, en combinaison avec du glyphosate ou un de ses sels, et/ou du glufosinate ou un de ses sels, et/ou du cinnamaldéhyde, et/ou *Bacillus subtilis,* et/ou *Gliocladium catenulatum.*

11. Composition selon l'une quelconque des revendications 1 à 8, comprenant un principe actif cosmétique.

12. Procédé de préparation d'une composition selon l'une quelconque des revendications 1 à 11, comprenant une étape de mélangeage d'au moins un ester méthylique ou éthylique d'acide gras, d'au moins un lipide de mannosylérythritol et d'au moins un tensioactif non-ionique de HLB supérieur ou égal à 12.

13. Emulsion comprenant une composition selon l'une quelconque des revendications 1 à 11 et de l'eau.

14. Procédé de préparation d'une émulsion selon la revendication 13, comprenant une étape de mélangeage d'une composition selon l'une quelconque des revendications 1 à 11 avec de l'eau.

15. Utilisation d'une composition selon l'une quelconque des revendications 1 à 8, en tant qu'adjuvant.

16. Utilisation selon la revendication 15, dans laquelle l'adjuvant possède une propriété mouillante et/ou pénétrante.

## Patentansprüche

1. Zusammensetzung, umfassend:

   - zwischen 75 und 99 Gew.-% mindestens eines Fettsäuremethyl- oder Fettsäureethylesters,
   - zwischen 0,01 und 20 Gew.-% mindestens eines Mannosylerythritollipids, und
   - zwischen 0,01 und 20 Gew.-% mindestens eines nichtionischen Tensids mit einem HLB-Wert von über oder gleich 12 (HLB - *hydrophilic-lipophilic balance*),

   wobei die Gewichtsprozentanteile in Bezug auf das Gesamtgewicht der Zusammensetzung angegeben sind.

2. Zusammensetzung nach Anspruch 1, umfassend zwischen 0,1 und 20 Gew.-% mindestens eines Alkohols in Bezug auf das Gesamtgewicht der Zusammensetzung, der eine Kohlenstoffatomanzahl zwischen 1 und 16 hat.

3. Zusammensetzung nach einem der Ansprüche 1 oder 2, wobei die Gesamtmenge an nichtionischem bzw. nichtionischen Tensid(en) mit einem HLB-Wert von über oder gleich 12 zwischen 0,5 und 10 Gew.-% in Bezug auf das Gesamtgewicht der Zusammensetzung beträgt.

4. Zusammensetzung nach einem der Ansprüche 1 bis 3, wobei die Gesamtmenge an MEL(s) zwischen 0,1 und 10 Gew.-% in Bezug auf das Gesamtgewicht der Zusammensetzung beträgt.

5. Zusammensetzung nach einem der Ansprüche 1 bis 4, mindestens zwei MELs umfassend, die aus der Gruppe ausgewählt sind, die aus MEL-A, MEL-B, MEL-C und MEL-D besteht.

6. Zusammensetzung nach einem der Ansprüche 1 bis 5, wobei der Fettsäuremethyl- oder Fettsäureethylester ein Fettsäuremethyl- oder Fettsäureethylester aus Raps-, Soja- und/oder Olivenöl ist.

7. Zusammensetzung nach einem der Ansprüche 1 bis 6, wobei das nichtionische Tensid mit einem HLB-Wert von über oder gleich 12 ein Fettsäure- und Polyethylenglycolester ist.

8. Zusammensetzung nach einem der Ansprüche 1 bis 7, mindestens eine freie Fettsäure und/oder mindestens ein Triglycerid umfassend.

9. Zusammensetzung nach einem der Ansprüche 1 bis 8, einen Pestizidwirkstoff umfassend.

10. Zusammensetzung nach einem der Ansprüche 1 bis 8, in Kombination mit Glyphosat oder einem seiner Salze und/oder Glufosinat oder einem seiner Salze und/oder Zimtaldehyd und/oder *Bacillus subtilis* und/oder *Gliocladium catenulatum.*

11. Zusammensetzung nach einem der Ansprüche 1 bis 8, einen Kosmetikwirkstoff umfassend.

12. Verfahren zur Herstellung einer Zusammensetzung nach einem der Ansprüche 1 bis 11, einen Schritt des Mischens mindestens eines Fettsäuremethyl- oder Fettsäureethylesters, mindestens eines Mannosylerithritollipids und mindestens eines nichtionischen Tensids mit einem HLB-Wert von über oder gleich 12 umfassend.

13. Emulsion, eine Zusammensetzung nach einem der Ansprüche 1 bis 11 und Wasser umfassend.

14. Verfahren zur Herstellung einer Emulsion nach Anspruch 13, einen Schritt des Mischens einer Zusammensetzung nach einem der Ansprüche 1 bis 11 mit Wasser umfassend.

15. Verwendung einer Zusammensetzung nach einem der Ansprüche 1 bis 8 als Zusatzstoff.

16. Verwendung nach Anspruch 15, wobei der Zusatzstoff eine befeuchtende und/oder eindringende Eigenschaft besitzt.

## Claims

1. Composition comprising:

    - between 75 and 99% by weight of at least one fatty acid methyl or ethyl ester,
    - between 0.01 and 20% by weight of at least one mannosylerythritol lipid, and
    - between 0.01 and 20% by weight of at least one non-ionic surfactant having an HLB value greater than or equal to 12,

    the percentages by weight being indicated with respect to the total weight of the composition.

2. Composition according to claim 1, comprising between 0.1 and 20% by weight of at least one alcohol having a number of carbon atoms comprised between 1 and 16, with respect to the total weight of the composition.

3. Composition according to any one of claims 1 or 2, in which the total quantity of non-ionic surfactant(s) having an HLB value greater than or equal to 12 is comprised between 0.5 and 10% by weight, with respect to the total weight of the composition.

4. Composition according to any one of claims 1 to 3, in which the total quantity of MEL(s) is comprised between 0.1 and 10% by weight, with respect to the total weight of the composition.

5. Composition according to any one of claims 1 to 4, comprising at least two MELs selected from the group constituted by MEL-A, MEL-B, MEL-C and MEL-D.

6. Composition according to any one of claims 1 or 5, in which the fatty acid methyl or ethyl ester is a methyl or ethyl ester of rapeseed, soya and/or olive oil fatty acid.

7. Composition according to any one of claims 1 to 6, in which the non-ionic surfactant having an HLB value greater than or equal to 12 is a polyethylene glycol fatty acid ester.

8. Composition according to any one of claims 1 to 7, also comprising at least one free fatty acid and/or at least one triglyceride.

9. Composition according to any one of claims 1 to 8, comprising a pesticide active ingredient.

10. Composition according to any one of claims 1 to 8, in combination with glyphosate or one of the salts thereof, and/or glufosinate or one of the salts thereof, and/or cinnamaldehyde, and/or *Bacillus subtilis,* and/or *Gliocladium catenulatum.*

11. Composition according to any one of claims 1 to 8, comprising a cosmetic active ingredient.

12. Process for the preparation of a composition according to any one of claims 1 to 11, comprising a step of mixing at least one fatty acid methyl or ethyl ester, at least one mannosylerythritol lipid and at least one non-ionic surfactant having an HLB value greater than or equal to 12.

13. Emulsion comprising a composition according to any one of claims 1 to 11 and water.

14. Process for the preparation of an emulsion according to claim 13, comprising a step of mixing a composition according to any one of claims 1 to 11 with water.

15. Use of a composition according to any one of claims 1 to 8, as adjuvant.

16. Use according to claim 15, in which the adjuvant has a wetting and/or penetrating property.

Fig. 1

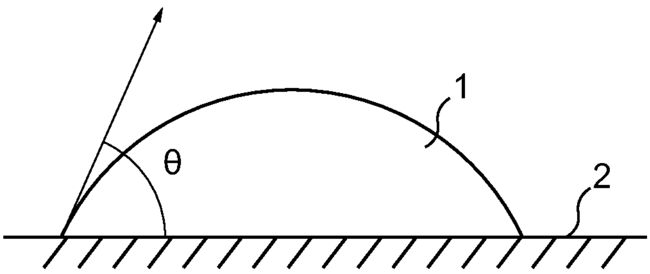


Fig. 2a

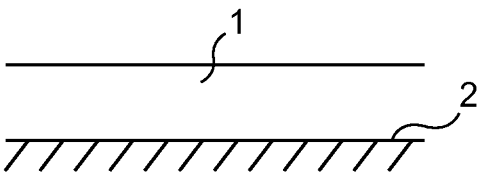


Fig. 2b

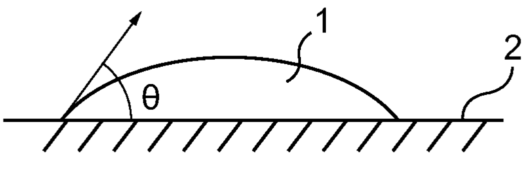


Fig. 2c

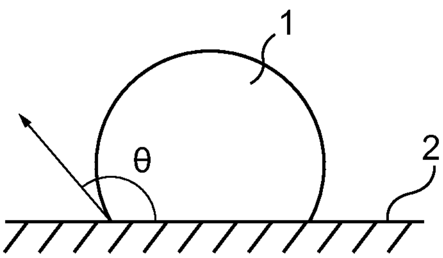

Fig. 3

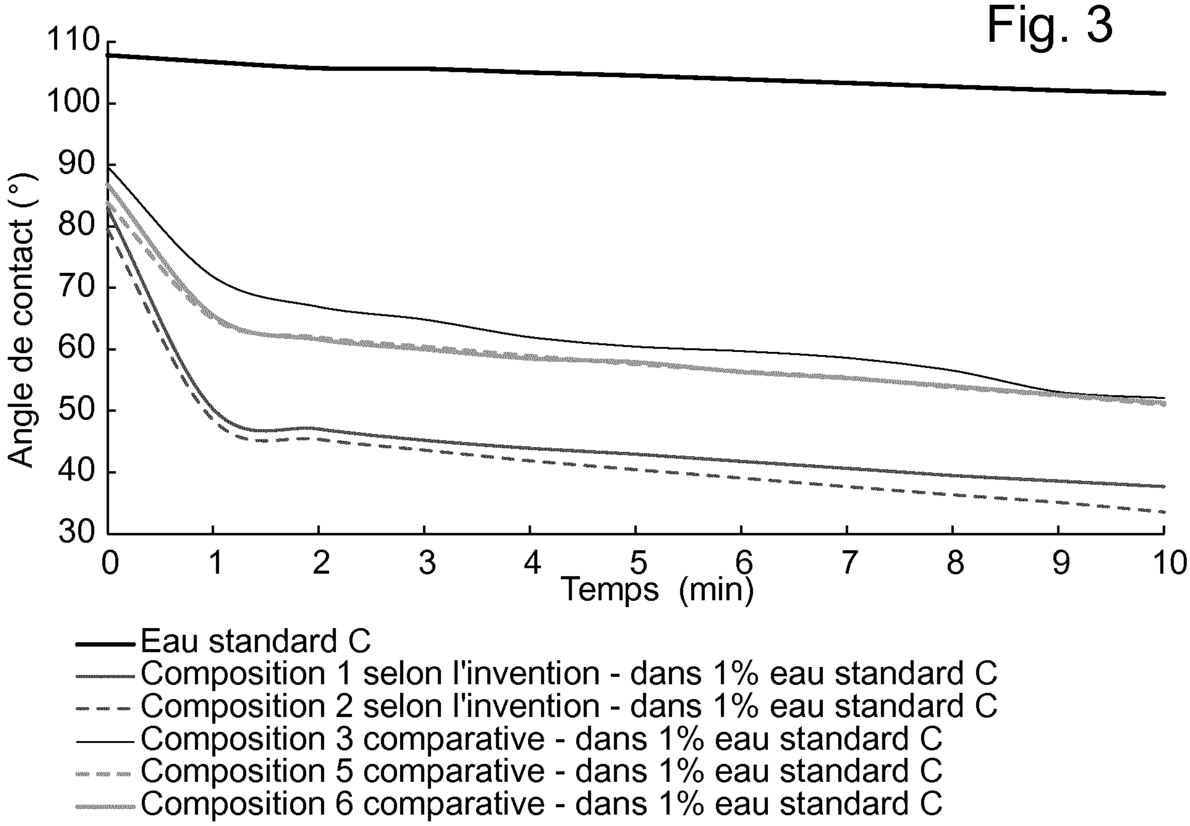
Angle de contact (°)
110
100
90
80
70
60
50
40
30
0
1
2
3
4
5
6
7
8
9
10
Temps (min)
Eau standard C
Composition 1 selon l'invention - dans 1% eau standard C
Composition 2 selon l'invention - dans 1% eau standard C
Composition 3 comparative - dans 1% eau standard C
Composition 5 comparative - dans 1% eau standard C
Composition 6 comparative - dans 1% eau standard C

Fig. 4

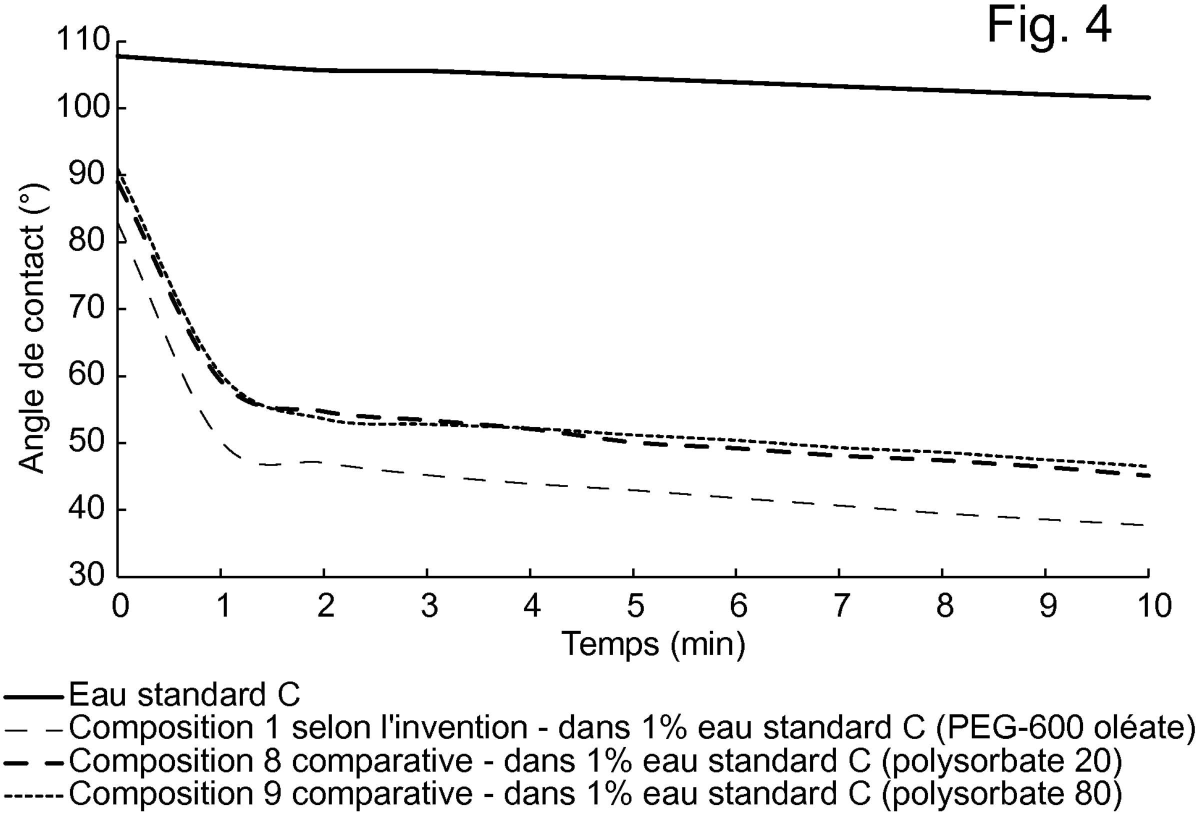
Angle de contact (°)
110
100
90
80
70
60
50
40
30
0
1
2
3
4
5
6
7
8
9
10
Temps (min)
Eau standard C
Composition 1 selon l'invention - dans 1% eau standard C (PEG-600 oléate)
Composition 8 comparative - dans 1% eau standard C (polysorbate 20)
Composition 9 comparative - dans 1% eau standard C (polysorbate 80)

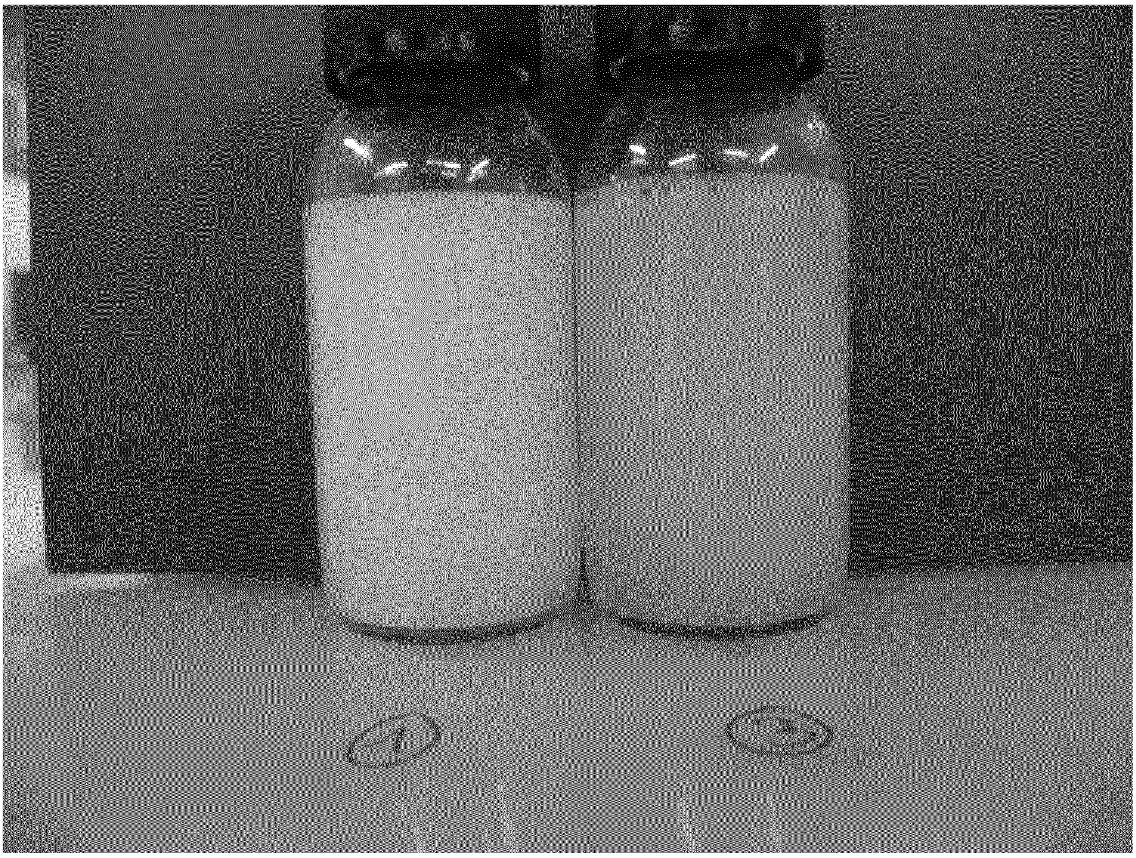
1
3

Fig. 5

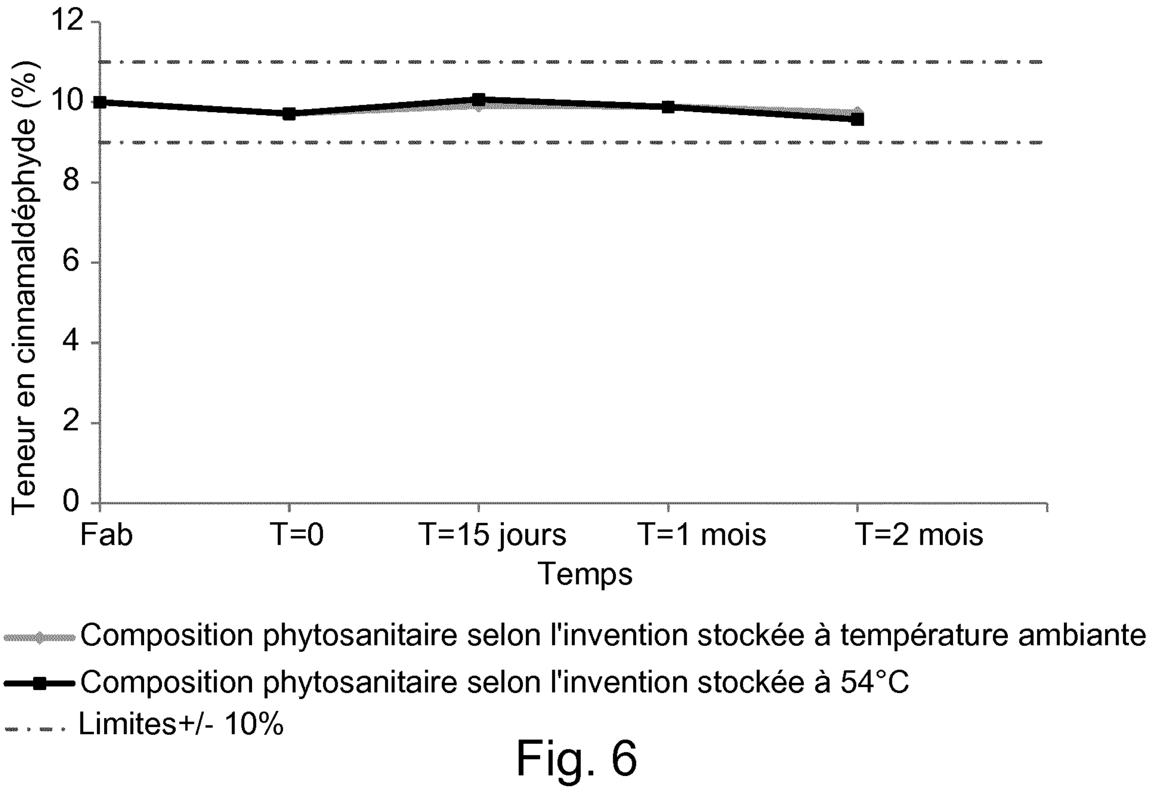
12
10
8
6
4
2
0
Teneur en cinnamaldéphyde (%)
Fab
T=0
T=15 jours
T=1 mois
T=2 mois
Temps
Composition phytosanitaire selon l'invention stockée à température ambiante
Composition phytosanitaire selon l'invention stockée à 54°C
Limites+/- 10%

Fig. 6

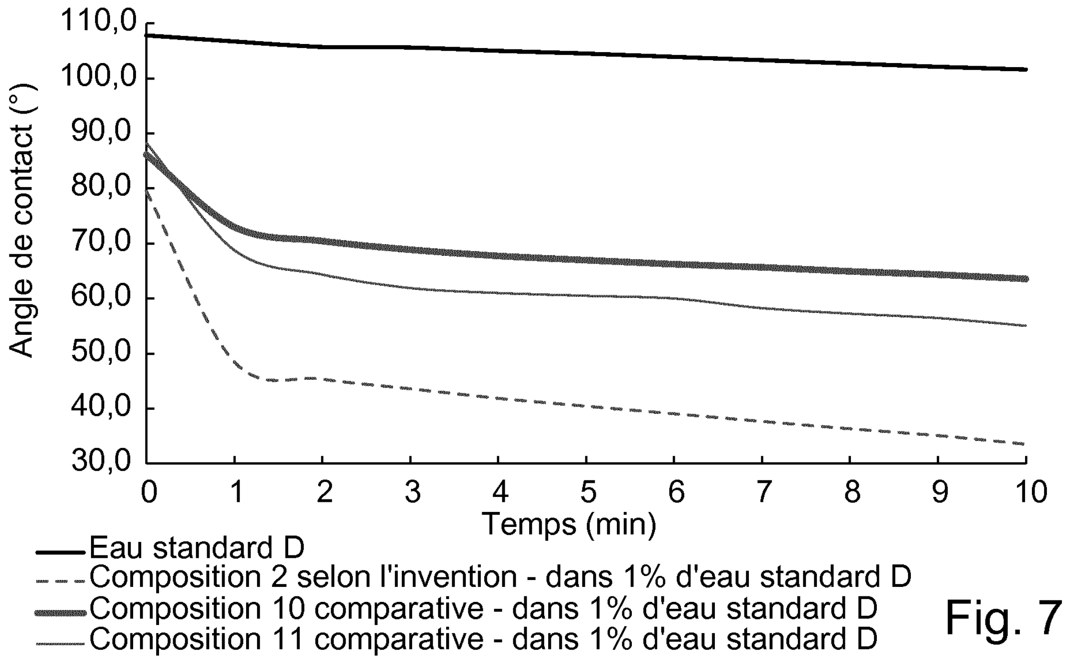


Fig. 7

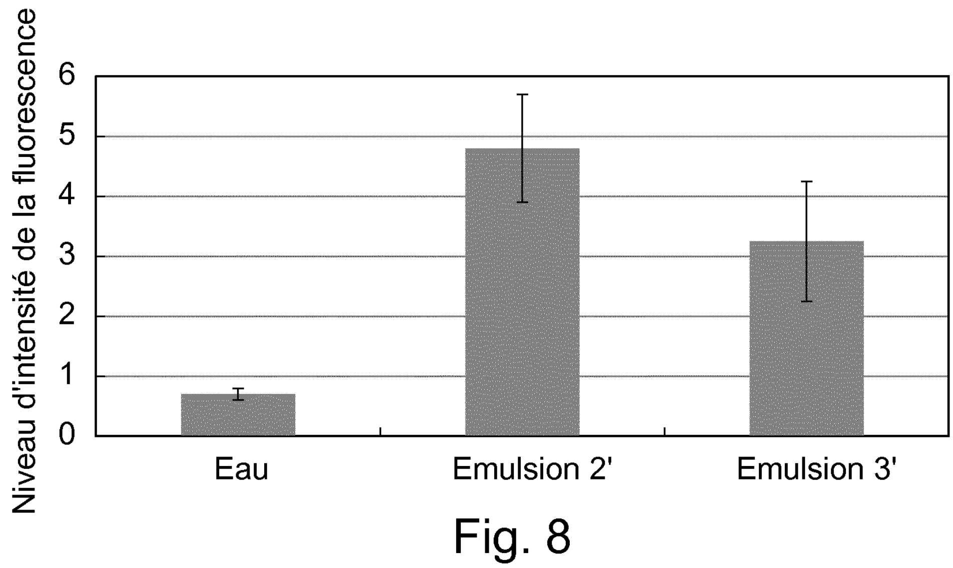


Fig. 8

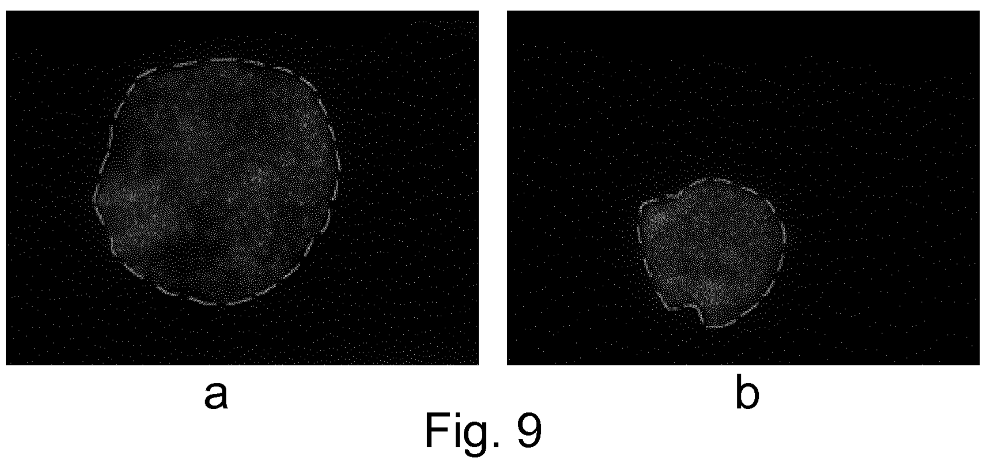


Fig. 9

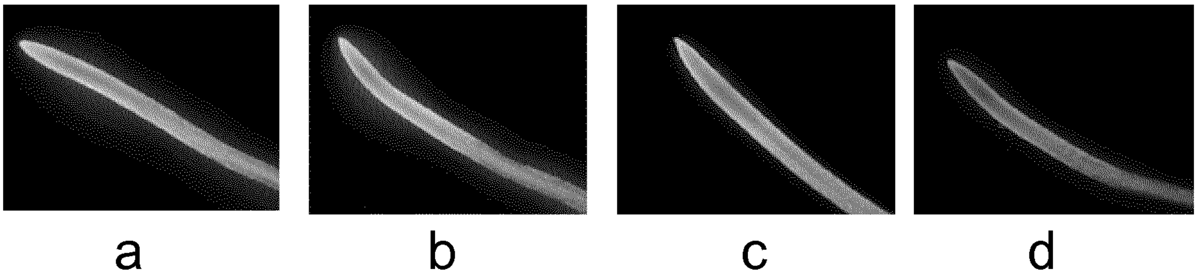


Fig. 10

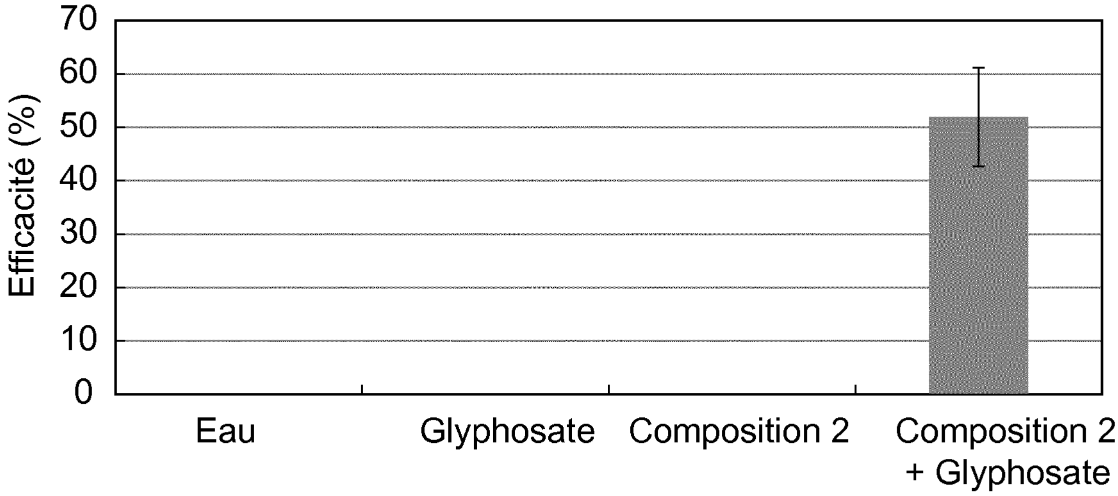


Fig. 11

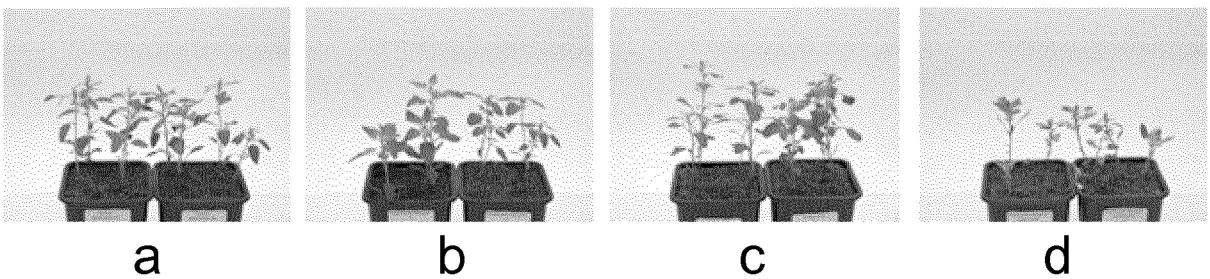


Fig. 12

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2013054194 A


### Littérature non-brevet citée dans la description


- **CHAIRMAN ; G MENSCHEL.** *CIPAC method 2000. Prepared by the German Formulation Panel (DAPF)*
- **RAU et al.** Downstream processing of mannosylerythritol lipids produced by Pseudozyma aphidis. *European Journal of Lipids Science and Technology,* 2005, vol. 107, 373-380